(19)

Europäisches Patentamt

European Patent Office

Office européen des brevets

(11) **EP 3 922 431 A1**

(12) **EUROPEAN PATENT APPLICATION**

(43) Date of publication:
**15.12.2021 Bulletin 2021/50**

(21) Application number: **20178818.9**

(22) Date of filing: **08.06.2020**

(51) Int Cl.:
**B29C 39/00** (2006.01)　　　**C12M 3/00** (2006.01)
**C12M 1/32** (2006.01)　　　**G01N 33/50** (2006.01)
**C12N 5/077** (2010.01)　　　**C12N 5/00** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Erasmus University Medical Center Rotterdam 3015 GE Rotterdam (NL)**
• **Academisch Ziekenhuis Leiden h.o.d.n. LUMC 2333 ZA Leiden (NL)**
• **Wageningen Universiteit 6708 PB Wageningen (NL)**

(72) Inventors:
• **IULIANO, Alessandro 3015 GE Rotterdam (NL)**

• **PIJNAPPEL, Wilhelmus Wenceslaus Matthias 3015 GE Rotterdam (NL)**
• **VAN DER PLOEG, Antje Tjitske 3015 GE Rotterdam (NL)**
• **SAGGIOMO, Vittorio 6707 JE Wageningen (NL)**
• **VAN DER WAL, Erik 2333 ZA Leiden (NL)**
• **DE GREEF, Jessica C. 2333 ZA Leiden (NL)**
• **VAN DER MAAREL, Silvère Maria 2333 ZA Leiden (NL)**

(74) Representative: **V.O. P.O. Box 87930 2508 DH Den Haag (NL)**

(54) **METHOD OF MANUFACTURING MICRODEVICES FOR LAB-ON-CHIP APPLICATIONS**

(57)　　A method of manufacturing a microstructure comprises printing a positive mold structure, filling the positive mold structure with a second material to form an elastically deformable negative mold structure, filling the negative mold structure with a third material to form the microstructure, and releasing the microstructure from the negative mold structure. Advantageously, the negative mold structure can be stretched to facilitate the release of the microstructure. For example, the microstructure comprises a chamber with capped micropillars for the generation and/or analysis of muscle tissue.

FIG 9

EP 3 922 431 A1

**Description**

TECHNICAL FIELD AND BACKGROUND

The invention relates to methods of manufacturing microdevices, in particular for lab-on-chip applications. The invention also relates to the use of such microdevices, e.g. in three-dimensional cell culture systems for growing muscle and other tissues. The invention also relates to the tissues generated using a lab-on-chip with such microdevices, and the use of such tissues e.g. for analysis and therapy.

The transition from two to three-dimensional (3D) engineered tissue cultures can change the way biologists perform in vitro functional studies. For example, 3D cell culture systems can be used to mimic the native structure and function of a tissue. Adopting this paradigm is particularly significant for contractile and load-bearing tissues, such as tendons, cardiac and skeletal muscle. For adequate lodging of 3D contractile tissues, flexible pillars are preferred to promote maturation and to provide tendon-like attachment points for contraction. Compared to alternative methods, such as micropatterned surfaces, flexible pillars offer functional advantages in the tracking of their displacement as a consequence of a tissue's contraction. Downscaling the size of engineered tissues is also possible, e.g., by producing pillars in a T-shape. For example, "caps" on top of each pillar can help the retention of smaller tissues under tension.

The production of microdevices for organ-on-chip applications is complicated and expensive. The main approaches used up to date are lithography, micro milling or laser etching, which require dedicated expertise and expensive facilities (e.g. clean rooms containing expensive machines). Moreover, these production systems lack flexibilities necessary for method development in biomedical applications to model organ and tissues in vitro. For example, when applied to the fabrication of a 3D culture device for engineered skeletal muscle, it is necessary to test different shapes and sizes.

Multistep photolithography can be used to generate negative molds for polydimethylsiloxane (PDMS) T-shaped pillars. For example, Legant et al. describe "Microfabricated tissue gauges to measure and manipulate forces from 3D microtissues" [Proc. Natl. Acad. Sci. 106, 10097 LP - 10102 (2009)]; Ramade et al. describe "Microfabrication of a Platform to Measure and Manipulate the Mechanics of Engineered Microtissues" [Methods Cell Biol. 121, 191-211 (2014)]; Kalman et al. describe "Quick and easy microfabrication of T-shaped cantilevers to generate arrays of microtissues" [Biomed. Microdevices 18, 43 (2016)]. However, the known approaches carry a limitation in the production of features larger than few hundreds of micrometers and lack versatility: producing a single master-mold is time-consuming and expensive, requiring most of the time dedicated facilities such as clean rooms.

To avoid multistep photolithography, molds can be generated using single-step lithography. For example, Agrawal et al. describe "Skeletal muscle-on-a-chip: an in vitro model to evaluate tissue formation and injury" [Lab Chip 17, 3447-3461 (2017)]; Osaki et al. describe "Microphysiological 3D model of amyotrophic lateral sclerosis (ALS) from human iPS-derived muscle cells and optogenetic motor neurons" [Sci. Adv. 4, eaat5847 (2018)]; Osaki et al. describe "On-chip 3D neuromuscular model for drug screening and precision medicine in neuromuscular disease" [Nat. Protoc. 15, 421-449 (2020)]. Also milled Teflon can be used. For example, Takahashi et al. describe "Engineered Human Contractile Myofiber Sheets as a Platform for Studies of Skeletal Muscle Physiology" [Sci. Rep. 8, 13932 (2018)]. However, these approaches are followed by manually gluing a thin square of PDMS on top of each pillar for obtaining the T-shape, making the methods time consuming and not suitable for high throughput.

Alternatively, a multiple step aluminum mold can be used to fabricate hammer-like PDMS pillars. For example, Ronaldson-Bouchard et al. describe "Engineering of human cardiac muscle electromechanically matured to an adult-like phenotype" [Nat. Protoc. 14, 2781-2817 (2019)]. In this case, high throughput can be obtained but at the expense of versatility, as each variation in size and shape of the pillars would require a new micro milled aluminum mold. As another alternative, hydrogel 3D printing can be used. [22] For example, Christensen et al. describe "3D Printed Hydrogel Multiassay Platforms for Robust Generation of Engineered Contractile Tissues" [Biomacromolecules 21, 356-365 (2020)]. However, this involves 3D printers that are expensive, difficult to operate, and not accessible for standard biomedical laboratories. As yet another alternative, Afshar et al. describe "A 96-well culture platform enables longitudinal analyses of engineered human skeletal muscle microtissue strength" [bioRxiv 562819 (2019) doi:10.1101/562819]. Here a set of devices containing small hook-shaped pillars has been created using a 3D printer. However, this method involves an expensive machine and has difficulties in particular when producing smaller structures. For example, Saggiomo et al. describe "Simple 3D Printed Scaffold-Removal Method for the Fabrication of Intricate Microfluidic Devices" [Advanced Science 2, 1500125 (2015)].

There is yet a need for improved methods of manufacturing and use of microdevices or chips with reproducible micron features for tissues cultures, which can be easily scaled up in a cost effective way. Ideally, these methods should be simple, versatile and applicable in standard biomedical laboratories independently of specific microfabrication expertise.

SUMMARY

Some aspects of the invention can be used to provide a fabrication pipeline based on affordable off the shelf 3D printers and novel replica molding strategies for rapid and easy in-house production of hundreds of 3D culture devices with customizable size and geometry. 3D printing offers the advantages of being inexpensive, easy to use, versatile, accessible to the biological researcher, it can be placed in any laboratory and it's fast both in the in-silico design and the physical fabrication. However, it has hitherto not been possible to generate very small devices, e.g. in polydimethylsiloxane (PDMS; a biocompatible material) or similar elastomers, with small geometric features that can support 3D tissue cultures by conventional replica molding. The current systems are based on the generation of stiff molds made of plastic or silicon, from which the final PDMS device is peeled off. This creates a limitation to the size and shape of the device, because small elements will be damaged upon the peeling process.

The fabrication pipeline described herein alleviates this problem using 3D printing to create the original structure and from that generating highly elastic molds made out of materials such as silicone elastomers, preferably low crosslinked siloxanes. One example of a suitable material, as used herein, is marketed under the name "Ecoflex™ 00-30" which can be obtained from the company "Smooth-On". Of course also other stretchable and/or soft materials can be used. Molds made from such materials can be stretched in order to provide an easy removal of the PDMS replica. This allows the preservation of extremely small features, including over-hanging parts, such as caps, which would be damaged with traditional systems. This allows, e.g., to create small devices that fit in a well of a 48- or 96 wells plate. The re-usability of the mold is also comparable to conventional systems, because the reuse of the molds allows a multiplication factor of at least fifteen, resulting in the generation of hundreds of devices from a single elastic mold. To clarify the principle of a mold: this is a template from which multiple replicas can be made. For example, one can use a negative structure, poor the material in liquid form, let it solidify, and obtain the final positive structure. To remove the structure from the negative mold, one can peel it off, or one can dissolve the negative mold. The disadvantage of peeling is that it may damage small structures. Using the stretchable material prevents this because it is flexible.

Advantages of the present method may include increased versatility compared to existing methods, as with our method sizes of, e.g., 0.25 mm can now be easily made in a standard laboratory on a bench. So far, similar sized devices could only be made using multi-step lithography-based methods in a clean room. For example, with our method we were able to form 0.5 mm thick pillars provided with over-hanging caps without damaging them in the fabrication process. To clarify why it is important to make these structures: to fit these in small wells of a 48 or 96 wells plate, one needs to make the devices smaller. There is a risk that the muscle tissue will slide off from the pillar, the caps will prevent this. The 3D printing approach offers a particular advantage when it comes to generating complex shapes in height. In this respect, it outperforms lithography. Potential areas of application may include organ-on-a-chip (for example cardiac muscle, tendons, or other contractile tissue), basic biological studies, 3D muscle-on-a-chip applications, drug screening, phase I-IV clinical trials, personalized medicine, et cetera.

In a preferred embodiment, a microstructure is manufactured as follows. A 3D printing process is used to form a positive mold structure of a printable, first material. For example, the positive mold structure is a positive of the microstructure to be manufactured. In other words, the microstructure will be a (positive) replica or exact copy of the printed structure. The positive mold structure can be casted or filled with a second material to form an elastically deformable negative mold structure. Advantageously, the second material forming the negative mold structure can be more flexible than (printed) first material forming the positive mold structure. For example, the second material (when forming the negative mold structure) has a lower Young's modulus than the first material (when forming the positive mold structure). Typically, the positive mold structure is casted or filled with the second material in liquid form, or at least softened form, wherein the second material is solidified (e.g. by curing) to form the stretchable negative mold structure. Once the second material is cured, it can be removed from the positive mold, forming, in this way, the negative mold. Next, the negative mold structure can be filled with a third material to form the microstructure replicas. Typically, the stretchable negative mold structure is filled with the third material in liquid form, or at least softened form, wherein the third material is solidified (e.g. by curing) to form the microstructure. Finally, the microstructure can be released from the negative mold structure. Advantageously, the negative mold structure is able to elastically deform, e.g. by stretching the mold, to facilitate the release of the microstructure including any sub-millimeter features and possibly overhanging protrusions such as described herein.

Preferably, the microstructure comprises at least one (vertical) micropillar with a first (maximum) diameter less than a millimeter, preferably less than 750 μm, e.g. between 100 - 500 μm, or even less. Advantageously, the inventors find that the high flexibility of the negative mold structure can enable the release of such thin structures without breaking. Typically, the micropillar has a length which is larger than its diameter, e.g. by at least a factor two, three, five, ten, or more. For example, the micropillar has a length between one and ten millimeters, or more, preferably between two and five millimeters.

More preferably, a top of the micropillar is provided with a cap having a second (maximum) diameter that is larger than the first diameter of the micropillar below by at least twenty percent (factor 1.2), fifty percent (factor 1.5), a factor two, or more, e.g. up to a factor three, four, or five. For example, the widened cap can serve to retain a tissue on the pillar, in particular when the pillar bends, e.g. due to contractile forces. Advantageously, the high flexibility of the negative mold structure can enable the material to elastically deform to accommodate an overhanging structure such as the cap, for releasing the micropillars even when the cap is relatively wide compared to the pillar diameter.

Most preferably, the micropillar comprises a widening section below the cap wherein a diameter of the widening section gradually decreases (over a slope) in a downward direction from the second diameter of the cap to the first diameter of the micropillar below. For example, the micropillar comprises a cylindrical section with a first diameter over a length of several millimeters followed by a (frustro)conical widening section over a length between 0.1 - 1 mm, preferably between 0.2 - 0.5 mm. Of course it will be understood that a cross-section of the pillar, cap, and/or widening section can be round, rectangular, or any other shape. Advantageously, the gradual change in diameter can help to release the capped micropillars from the stretchable (negative) mold structure, e.g. without the caps breaking off. For example, the widening section makes an obtuse angle of more than ninety degrees with respect to a length of the pillar below, preferably more than hundred degrees, more preferably at least hundred twenty degrees, or even more than hundred forty degrees.

Typically, the microstructure comprises a set of two or more micropillars. Most preferably the one or more micropillars are disposed in a microchamber. For example, the microchamber is configured to form (micro)volume for holding a relatively small amount liquid, e.g. with a maximum capacity between 0.1 - 100 $\mu$l (microliter), preferably between 1 - 50 $\mu$l, most preferably between 5 - 25 $\mu$l. In some embodiments, the printed mold structure comprises a plurality of microchambers. For example, a single mold can be used to manufacture ten, fifteen, twenty, or more microchambers with respective micropillars at the same time. Furthermore, the elastically deformable negative mold structure can be reused to sequentially create multiple times the same microstructures, each with a plurality of microchambers. Furthermore, the printed positive mold structure can be reused to create multiple elastically deformable negative mold structures. So it will be appreciated that with a single printing process microstructures, e.g. microchambers, can already be mass-produced.

In some embodiments, the second material forming the stretchable negative mold structure comprises or essentially consists of a first elastomer, most preferably a silicone elastomer such as Ecoflex. An elastomer is a polymer with viscoelasticity, e.g. possessing both viscosity and elasticity, and typically has very weak intermolecular forces, generally low Young's modulus and high failure strain compared with other materials. Elastomers are usually thermosets but may also be thermoplastic. The long polymer chains cross-link during curing, i.e., vulcanizing. The elasticity is derived from the ability of the long chains to reconfigure themselves to distribute an applied stress. The covalent cross-linkages ensure that the elastomer will return to its original configuration when the stress is removed. As a result of this extreme flexibility, elastomers can reversibly extend from 5-700%, depending on the specific material. Thermoplastic elastomers (TPE), sometimes referred to as thermoplastic rubbers, are a class of copolymers or a physical mix of polymers (usually a plastic and a rubber) that consist of materials with both thermoplastic and elastomeric properties. TPEs are relatively easy to use in manufacturing, for example, by injection molding. Temperature effects are also present in the demonstrated elasticity of a polymer. Elastomers that have cooled to a glassy or crystalline phase will have less mobile chains, and consequentially less elasticity, than those manipulated at temperatures higher than the glass transition temperature of the polymer.

In some embodiments, the third material forming the microstructure comprises or essentially consists of a different, second elastomer. For example, the microstructure is formed of a (biocompatible) elastomer, preferably a polymeric organosilicon compound, most preferably polydimethylsiloxane (PDMS). PDMS is viscoelastic, meaning that at long flow times or high temperatures, it acts like a viscous liquid, similar to honey. However, at short flow times or low temperatures, it acts like an elastic solid, similar to rubber. For example, the PDMS is applied in liquid form to the negative mold structure, and cured to form the microstructure.

Preferably, the second material to form the stretchable negative mold structure is the most flexible of the three materials so that this second material, when formed, can easily release the microstructure. For example, the second material forming the stretchable negative mold structure is more flexible than the first material forming the 3D printed positive mold structure, e.g. having a Young's modulus that is at least ten percent lower (at the same temperature), preferably at least fifty percent lower, or even lower, e.g. by at least a factor two, three, five, ten, or more.

In some embodiments, the second material forming the stretchable negative mold structure is capable of being reversibly (elastically) stretched (along at least one dimension) by at least a factor two without breaking, preferably at least a factor three, five, seven, nine, or more. In other or further embodiments, the second material forming the stretchable negative mold structure is also relatively soft, e.g. having a Shore Hardness (e.g. using standard test ASTM D-2240) softer than Shore 00-60, preferably softer than Shore 00-50, more preferably softer than Shore 00-40, most preferably softer than Shore 00-30, e.g. down to a softness of Shore 00-10, or softer. In other or further embodiments, the second material forming the stretchable negative mold structure can be relatively easily stretched, e.g. having a 100% Modulus (force for 100% elongation, e.g. using standard test ASTM D-412) less than 0.1 MPa (15 psi), preferably less than 0.07 MPa (10 psi), e.g. down to 0.05 MPa (7 psi), or less. Alternatively, or additionally, the second material forming the stretchable negative mold structure preferably has a tensile strength (e.g. using standard test ASTM D-412) less than 2.5 MPa, more preferably less than 1.5 MPa, e.g. down to 0.5 MPa, or less. For some applications the third material forming the microstructure can also possess at least some flexibility, e.g. to provide resilience against a force exerted by muscular tissue, although this third material is typically (much) less flexible than the second material forming the stretchable negative mold. For example, the second material forming the stretchable negative mold structure is more flexible than the third material forming the microstructure, e.g. having a Young's modulus that is at least ten percent lower (at the same temperature), preferably at least fifty percent lower, or even lower, e.g. by at least a factor two, three, five, ten, or more.

For some applications the first material forming the 3D printed positive mold structure can be relatively rigid or hard. This may allow a most faithful copying and optional re-use of this structure. For example, third material forming the microstructure is more flexible than the first material forming the 3D printed positive mold structure, e.g. having a Young's modulus that is at least ten percent lower (at the same temperature), preferably at least fifty percent lower, or even at least a factor two, three, five, ten, or more lower.

In a preferred embodiment, the 3D printing process comprises stereo-lithography (SLA printing), masked stereo-lithography (mSLA) or digital light processing (DLP). For example, the first material comprises a liquid polymeric resin that is solidified by a laser spot or any other light pattern, e.g. by a (UV) light source projected via an LCD mask or any other digital light projector. Preferably, the liquid polymeric resin is composed of acrylates. In some embodiments, both the 3D printed positive mold structure and the elastically deformable negative mold structure are subjected to a coating, which facilitates the unmolding process by making their respective surfaces inert. For example, the coating comprises perfluorodecyltrichlorosilane (PFOTS).

In some embodiments, the microstructure is part of a lab-on-chip (e.g. muscle-on-chip) design. Accordingly, some aspects of the invention can be embodied as a lab-on-chip comprising microstructure with one or more microchambers obtainable by the methods described herein. In a preferred embodiment a respective microchamber of the one or more microchambers with a respective maximum capacity for holding a small amount of liquid, e.g., between 5 - 100 $\mu$l. Most preferably, the respective microchamber comprises at least two (flexible) micropillars. For example, a respective micropillar of the at least two micropillars has a first diameter less than a millimeter, preferably less than 750 $\mu$m, wherein a top of the respective micropillar is provided with a cap having a second diameter that is larger than the first diameter of the micropillar below, wherein the respective micropillar comprises a widening section below the cap wherein a diameter of the widening section gradually decreases in a downward direction from the second diameter of the cap to the first diameter of the micropillar below. When manufactured according to the present methods, the micropillars including the cap and widening section there between are integrally formed of an elastomeric material such as PDMS. Advantageously, the integrally micropillars can be integrally formed without glue between the caps and pillars.

Some aspects of the invention can also be embodied in a stereo-lithography apparatus, e.g. comprising a memory storing program instructions that, when executed causes the apparatus to print a positive mold of the lab-on-chip as described herein. Other or further aspects can be embodied as a non-transitory computer-readable medium storing program instructions that when executed by the stereo-lithography apparatus, causes the apparatus to print the positive mold of the lab-on-chip.

Aspects of the invention can also relate to the use of a lab-on-chip or other microstructures, e.g. as described herein, for generating and/or analyzing a contractile and load-bearing tissue, such as tendons or muscle tissue, preferably 3D skeletal muscle tissue. For example, the analyzing comprises determining a contraction of the tissue against a flexing of the micropillars.

Conventionally, in vitro generation of 3D skeletal muscle tissue can use, as cell source muscle, stem cells isolated from muscle biopsies or via differentiation of pluripotent stem cells (embryonal or induced pluripotent stem cells, ESCs or iPSCs, respectively). Muscle stem cells isolated from muscle biopsies can give high quality 3D skeletal muscle tissue, but a disadvantage is that these cells are rare, need to be expanded, and rapidly lose their capacity to proliferate and differentiate into muscle fibers upon expansion. ESCs or iPSCs can be used as alternative cell source following differentiation in vitro into muscle progenitors. 3D skeletal muscle tissue can be made e.g. using ESC or iPSC-derived muscle progenitors using an overexpression system involving overexpression of the master myogenic transcription factors Pax7 or MyoD. These tissues have moderate quality in terms of contractile force and the development of aligned myofibers. 3D skeletal muscle tissue derived from myogenic progenitors made from iPSCs in a transgene-free manner has not been reported so far.

One problem is that human 3D muscle cultures are generated using cells obtained after overexpression of transcription factors, resulting in artificial muscle cells, or using cells obtained from muscle biopsies, which lose their capacity to proliferate and differentiate after only few passages. Human 3D muscle cultures that have been generated in a transgene-free manner (using small molecules) from cells that retain the capacity to proliferate and differentiate upon passaging and that show high quality, as indicated by long, aligned muscle fibers, no large empty spaces between the fibers, expression of sarcolemmal (=plasma membrane) markers such as dystrophin around the muscle fibers, and high contractile force, have not been reported to date. Such muscle bundles are desirable because they are not derived from artificial overexpression but from the activation of signaling pathways using small molecules that mimic natural embryonic development of pluripotent cells to muscle stem cells. In addition, it is a challenge to find optimal experimental conditions for the generation of high quality human 3D muscle bundles.

With the present teachings, we can use expandable human skeletal muscle derived from iPSCs in transgene-free manner and find culture conditions that enable the generation of high-quality 3D skeletal muscle tissue. This is indicated e.g. by the generation of functional sarcomeres (which are the contractile units of a muscle cell), high level of fiber alignment, and the highest contractile force in vitro reported to date. In these aspects, our 3D muscle cultures closely mimic the architecture of real muscle cells. Optimal experimental conditions defined in this application include an optimal cell density, hydrogel and culture medium composition and adequate geometry of the culturing device. So our 3D model of human skeletal muscle tissue is superior to existing models because 1) it is based on myogenic progenitors obtained from human iPS cells in a transgene-free manner; 2) it shows the highest contractile force and best maturation reported to date for iPSC-derived, transgene-free 3D muscle tissue models.

A further advantage of the methods of the invention is that it has become possible to isolate muscle stem cells. Muscle stem cells are stem cells that are present in skeletal muscle tissue, that can self-renew and can give rise to skeletal muscle cells. Upon isolation of muscle stem cells from donor muscle tissue, the muscle stem cells lose their stem cell character, meaning that they lose their capacity to give rise to new skeletal muscle cells. Before the present invention, it has not been possible to set appropriate culturing conditions suitable to expand human muscle stem cells in vitro while retaining their stem cell character. The present inventors surprisingly found that the use of the lab-on-chip according to the invention, in combination with the culture conditions described herein, allow the generation and expansion of muscle stem cells within generated skeletal muscle bundles. These muscle stem cells have the potential to be used in regenerative approaches, e.g. after injury or in muscle disease to regenerate damaged muscle tissue. For instance, muscle stem cells expanded in vitro with a method according to the invention for generating muscle tissue, as detailed herein below, can be engrafted in patients with muscle disorders or suffering from muscle injury to regenerate muscle tissue that has been lost.

Other or further aspects of the invention may thus relate to the use of a microstructure, e.g. manufactured according to the methods described herein. In a preferred embodiment, a method for generating muscle tissue comprises providing myogenic progenitor cells, a hydrogel and culture medium to the microstructure, e.g. providing this in a chamber with micropillars as described herein, and culturing the myogenic progenitor cells to generate muscle tissue.

The muscle tissue is preferably skeletal muscle tissue. The muscle tissue is further preferably 3-dimensional (3D) muscle tissue. In a particularly preferred embodiment, the muscle tissue is 3D skeletal muscle tissue. Skeletal muscle tissue consists of skeletal muscle cells, which are also referred to as skeletal muscle fibers. These skeletal muscle fibers are joined together to form skeletal muscle bundles. Hence, in a preferred embodiment the skeletal muscle tissue, in particular 3D skeletal muscle tissue, comprises or consists of skeletal muscle bundles. In another preferred embodiment, the muscle tissue is muscle stem cells. The microstructures as described herein are particularly suitable for the generation of 3D skeletal muscle bundles, as demonstrated in the examples herein. It is further demonstrated in the examples that muscle stem cells can be isolated from these skeletal muscle bundles, which retain their capacity to give rise to skeletal muscle cells. The use of the microstructure as described herein, preferably in combination with culture conditions described herein, enable the generation of high-quality 3D skeletal muscle bundles. This is demonstrated by the generation of functional sarcomeres (the contractile units of a muscle cell), a high level of fiber alignment, and a highest contractile force as determined *in vitro* reported to date. Also provided by the invention is therefore muscle tissue obtainable by a method as described herein. Said muscle tissue is preferably 3D skeletal muscle tissue, more preferably skeletal muscle bundles. In another preferred embodiment, said muscle tissue is muscle stem cells. Said muscle tissue is further preferably human muscle tissue, in particular human skeletal muscle bundles or human muscle stem cells. As demonstrated in the examples herein, skeletal muscle bundles that have a specific twitch force *in vitro* of 12nM/mm$^2$ and a specific tetanus force of 35 mN/mm$^2$ have been obtained with a method according to the invention. Therefore, said muscle tissue, in particular skeletal muscle bundles, according to the invention preferably has a specific twitch force *in vitro* of more than 7 mN/mm$^2$, and a specific tetanus force of more than 33 mN/mm$^2$ as determined by video imaging analysis, as described in Legant et al. "Microfabricated tissue gauges to measure and manipulate forces from 3D microtissues", PNAS (25), 10097-10102 (2009). Said specific twitch force is preferably more than 8 mN/mm$^2$, more preferably more than 9 mN/mm$^2$, more preferably more than 10 mN/mm$^2$, more preferably more than 11 mN/mm$^2$, and said specific tetanus force is preferably more than 34 mN/mm$^2$, e.g. specific twitch force *in vitro* of about 12 mN/mm$^2$, and a specific tetanus force of about 35 mN/mm$^2$.

In this video imaging analysis method, twitch and tetanic contractions are induced by electrical stimulation with either a frequency of 1 Hz (twitch) or 20 Hz (tetanus) at 2.5 V/cm with 10% duty cycle using a function generator. During stimulation the pillar displacement is captured with a fast camera recording at 60 frames per second. The height of the TESM on the pillar is measured for each replica and included in the calculation. Contractile force can be calculated using Young's Elastic Modulus (E) of PDMS, the geometrical properties and the position of the TESM on the pillar (R, a and

L) and displacement ($\delta$) of the PDMS pillar using the following *Force in* $N = \frac{6E\pi R^4}{4a^2(3L-a)}\delta$ formula [Legant et al. "Microfabricated tissue gauges to measure and manipulate forces from 3D microtissues", PNAS (25), 10097-10102 (2009)]. For the calculation of specific force paraffin based cross sections can be generated from e.g. 2-3 TESMs and the specific force can be calculated by dividing the force by cross sectional area.

As used herein the term "myogenic progenitor cells" refers to cells that are not yet fully or terminally differentiated, but during further differentiation give rise to or forms muscle cells, preferably skeletal muscle cells or skeletal muscle fibers. These skeletal muscle fibers can be joined under appropriate culturing conditions to form 3D skeletal muscle bundles. In a preferred embodiment, the myogenic progenitor cells are human myogenic progenitor cells. However, for applications for other animals non-human myogenic progenitor cells can be used, preferably of the same species as the animal that will be treated.

Myogenic progenitor cells can for instance be obtained by differentiation of pluripotent stem cells, such as embryonal stem cells (ESCs) or induced pluripotent stem cells (iPSCs). In a preferred embodiment, the myogenic progenitor cells are obtained by differentiation of iPSCs, more preferably human iPSCs. As used herein the term "iPSCs" refers to stem cells that are produced from differentiated adult, neonatal or fetal cells that have been induced into cells capable of differentiating into tissues of all three germ or dermal layers: mesoderm, endoderm, and ectoderm. Methods of producing PSCs, such as e.g. iPSCs, are well known in the art, and for instance described in detail in U.S. Pat. Nos. 8,058,065; 8,129,187; 8,211,697; 8,257,941; 8,278,104; 8,546,140; 8,791,248; 9,175,268; U.S. Patent Publn. Nos. 2008/0003560; 2008/0233610; 2010/0184227; 2013/0029866; 2013/0040302; 2013/0130387; EP 2 072 618 A1; WO/2009/032194; WO/2009/032456; WO 2010/042490; and WO 2010/077955, which are incorporated herein by reference.

ESCs or iPSCs can be differentiated into myogenic progenitor cells using an overexpression system involving overexpression of myogenic transcription factors, in particular Pax7 or MyoD. Alternatively, ESCs or iPSCs can be differentiated into myogenic progenitor cells in a transgene-free manner, using appropriate culture medium. The myogenic progenitor cells are preferably transgene-free. "Transgene-free" as used herein means that the cells do not contain heterologous nucleic acid sequences, such as genes encoding proteins or parts thereof. A suitable method to obtain transgene-free myogenic progenitor cells from PSCs, preferably iPSCs, is described in WO 2017/196175, which is incorporated herein by reference. The method described WO 2017/196175 comprises the steps of:

a) providing pluripotent stem cells (PSCs), preferably iPSCs;
b) culturing the PSCs in a synthetic culture medium supporting differentiation of said PSC towards a myogenic cell lineage for (i) a first period of 3-8 days in the presence of between 2-5 microM, preferably about 3.5 microM, of CHIR99021, (ii) a second period of 5-20 days in the presence of 10-30 ng/ml of FGF2; and, optionally, (iii) a third period of 10-20 days in the presence of insulin-transferrin-selenium-ethanolamine (ITS-X), to thereby provide a cell culture of pre-differentiated PSCs comprising myogenic progenitors cells;
c) isolating from the cell culture comprising myogenic progenitors cells C-Met+ and Hnk1- myogenic progenitor starting cell (s), to thereby provide a purified myogenic cell lineage;
d) expanding the isolated C-Met+ and Hnk1- myogenic progenitor starting cell in a synthetic culture medium comprising fetal bovine serum (FBS), preferably in a concentration of about 10% (w/w), and 90-110 ng/ml of FGF2 for at least 1 passage, preferably at least 7 passages, to thereby provide a cell culture comprising a population of expanded C-Met+ and Hnk1- myogenic progenitor cells. It is preferred that the step of expanding said at least one C-Met+ and Hnk1- myogenic progenitor starting cell in step d) is performed in a culture medium comprising a ROCK inhibitor during at least the cultivation prior to the first passage.

The culture medium is preferably at least in part in the form of a hydrogel. The culture medium preferably comprises a combination of a hydrogel and a liquid culture medium, in particular a proliferation medium and/or differentiation medium as detailed herein below. Preferably the cells are first cultured in the presence of the proliferation medium, e.g. for about 2 days, and subsequently in the present of the differentiation medium, e.g. for 5-10, preferably 6-8 days.

The hydrogel is preferably composed of fibrinogen, preferably bovine fibrinogen, a hydrogel system and myogenic progenitor proliferation medium, and optionally comprises additional components. The fibrinogen is preferably dissolved in culture medium such as DMEM, e.g. DMEM high glucose. The fibrinogen is preferably present in a concentration of 0.2-4 mg/ml, preferably 0.5-2 mg/ml, such as about 1 mg/ml, based on the total volume of the hydrogel. The hydrogel system is preferably extracellular matrix proteins, such as laminin, nidogen, collagen, elastin, fibronectin and/or heparan sulfate proteoglycans. Suitable extracellular matrix protein mixtures are for instance Matrigel® or Matrigel growth factor reduced® (Corning Life Sciences), Cultrex® BME (R&D Systems) or Geltrex® Matrix (Thermo Fisher Scientific). The hydrogel system is preferably present in an amount of 15-30% w/w, preferably 17-25% w/w, such as about 20% w/x, based on the total weight of the hydrogel. Suitable myogenic progenitor proliferation medium is for instance DMEM high glucose supplemented with Penicillin/Streptomycin/Glutamine (e.g. 100 U/ml), fetal bovine serum (e.g. 10% w/w), FGF2 (e.g. 50-200 ng/ml, preferably 80-120 ng/ml, more preferably about 100 ng/ml). The myogenic progenitor proliferation medium is preferably present in an amount of 60-85% v/v, more preferably about 65-75% v/v, based on total volume of the hydrogel. It preferably forms the remainder of the total volume of the hydrogel, in addition to the fibrinogen, optionally dissolved in culture medium such as DMEM, and the extracellular matrix proteins. The fibrinogen, the hydrogel system and the myogenic progenitor proliferation medium are mixed in concentrations sufficient to allow hydrogel formation, which can be readily assessed by a person skilled in the art. For instance, in the examples described herein bovine fibrinogen is present in a final concentration of e.g. 0.5 - 2 mg/ml (preferably about 1 mg/ml), the extracellular matrix proteins are present in a concentration of 20% v/v based and the myogenic progenitor proliferation medium in a concentration of 69% v/v, both based on the total volume of the hydrogel. The hydrogel preferably further comprises thrombin, e.g. from human plasma, such as in a concentration of 0.5-5% v/v, preferably 0.5-2.5% v/v, such as about 1% v/v, based on the total volume of the hydrogel.

The, hydrogel, culture medium and myogenic progenitor cells can be added to the chambers of the microstructure together or sequentially. Preferably, the myogenic progenitor cells are mixed with the components of the culture medium, in particular components of the hydrogel, and the mixture is added to the microstructure, where the myogenic progenitor cell containing hydrogel is formed. Myogenic progenitor cells are preferably added to the chambers of the microstructure at a concentration of $10^{\wedge}6 - 10^9$ cells/ml, more preferably at a concentration of $10^7 - 10^8$ cells/ml, based on the total amount of the hydrogel and culture medium together. Preferably, a total amount of $2 \times 10^5 - 1 \times 10^6$ myogenic progenitor cell is added to a chamber of preferably 15, 30 or 50 $\mu$l. Suitable culture conditions, including the composition of the hydrogel and cell density of the myogenic progenitor cells is provided in the examples. Preferably, TESM proliferation medium (e.g. myogenic progenitor proliferation medium as described above supplemented with 6-aminocaproic acid, preferably in a concentration of 0.5-5 mg/ml, more preferably 0.8-2.5 mg/ml (e.g. 1.5 mg/mL), or aprotinin, preferably in a concentration of 60-100 $\mu$g/ml, more preferably 70-90 $\mu$g/m (e.g. 80 $\mu$g/ml) based on total volume of the proliferation medium, is added to the chambers of the microstructure containing myogenic progenitor cells and hydrogel. Preferably, 6-aminocaproic acid is used. Optionally the chambers of the microstructures are coated prior to addition of the myogenic progenitor cell containing hydrogel to reduced adhesion of the hydrogel, for instance with Pluronic, e.g. F-127 (e.g. Sigma-Aldrich). The TESM proliferation medium can be replaced by TESM differentiation medium to initiate differentiation of the myogenic progenitor cells, e.g. after approximately 2 days. Preferably the differentiation medium, such as DMEM or DMEM high glucose, preferably DMEM high glucose, comprising antibiotics, ITS-X, preferably in a concentration of 0.5-2.5% v/v, knock-out serum replacement, preferably in a concentration of 0.5-2.5% v/v, L-glutamine and 6-aminocaproic acid, preferably in a concentration of 0.5-5 mg/ml, more preferably 0.8-2.5 mg/ml (e.g. 1.5 mg/mL), or aprotinin, preferably in a concentration of 60-100 $\mu$g/ml, more preferably 70-90 $\mu$g/m (e.g. 80 $\mu$g/ml). Preferably, 6-aminocaproic acid is used. Suitable TESM differentiation medium is for instance DMEMhigh glucose supplemented with 1% knock-out serum replacement, 1% ITS-X, 1% penicillin-G, 1% Glutamax, and 6-aminocaproic acid (1.5 mg/mL). The cells are subsequently cultured in the presence of TESM differentiation medium until the skeletal muscle tissue, in particular 3D skeletal muscle bundles, is obtained, e.g. for 5-10 days, such as 6, 7 or 8 days. It is further preferred that the culturing is performed under agitation of the microdevice, e.g. at 50-60 rpm. Further details and preferred culture conditions are provided in the examples herein. In a preferred embodiment, the method further comprises a step of isolating the muscle tissue from the microdevice. In one embodiment, skeletal muscle bundles are isolated. In another embodiment, muscle stem cells are isolated.

The muscle tissue generated with a method as described herein has a wide variety of applications. For instance, the muscle tissue can be used as skeletal muscle tissue model for biological studies, drug screening, and in therapy. Also provided is therefore muscle tissue generated by a method for generating muscle tissue according to the invention. Said muscle tissue is preferably 3D skeletal muscle tissue, more preferably skeletal muscle bundles, or muscle stem cells. In one embodiment said muscle tissue is comprised in a lab-on-chip and/or microstructure according to the present invention.

Also provided is therefore a use of muscle tissue as described herein, preferably skeletal muscle bundles, as a model for drug testing, in particular for the *in vitro* screening of a test compound or drug.

The muscle tissue, preferably skeletal muscle tissue or muscle stem cells, is for instance suitable for use in regenerative therapy, in particular regenerative skeletal muscle therapy, which refers to the process of replacing or regenerating skeletal muscle cells or tissues to restore or establish normal functioning thereof. Treatment of a muscle disorder and regenerative skeletal muscle therapy can for instance be used in the treatment of congenital muscle diseases or congenital muscular dystrophy (CMD), such as Duchenne and Becker muscular dystrophy, or to regenerate injured skeletal muscle tissue following trauma. Also provided is therefore muscle tissue as described herein, preferably 3D skeletal muscle tissue, more preferably skeletal muscle bundles, or muscle stem cells, for use in therapy, including regenerative therapy, in particular regenerative skeletal muscle therapy. Also provided is muscle tissue as described herein, preferably 3D skeletal muscle tissue, more preferably skeletal muscle bundles, or muscle stem cells, for use in the treatment of a muscle disorder. Also provided is a use of muscle tissue as described herein, preferably 3D skeletal muscle tissue, more preferably skeletal muscle bundles, or muscle stem cells, in the manufacture of a medicament for regenerative therapy, in particular regenerative skeletal muscle therapy, and/or for treatment of a muscle disorder. Also provided is a method for regenerative therapy, in particular regenerative skeletal muscle therapy, and/or treatment of a muscle disorder in a subject in need thereof, comprising administering to the subject a therapeutically effective amount of muscle tissue as described herein, preferably 3D skeletal muscle tissue, more preferably skeletal muscle bundles, or muscle stem cells. The regenerative therapy, in particular regenerative skeletal muscle therapy, is preferably used in the treatment of a congenital muscle disease or congenital muscular dystrophy, or to regenerate injured skeletal muscle tissue following trauma. In regenerative therapy, said muscle tissue is preferably muscle stem cells. The term "muscle disorder", as used herein, refers to a disease or disorder that affects the muscle, preferably skeletal muscle, system. Such disorders include muscular dystrophy including Duchenne muscular dystrophy, Becker muscular dystrophy, myotonic dystrophy, limb-girdle dystrophy and facioscapulohumeral dystrophy; congenital muscular dystrophies; congenital myopathies; distal myopathies; myotonic syndromes; ion channel muscle diseases; malignant hyperthermias; metabolic myopathies; hereditary cardiomyopathies; congenital myasthenic syndromes; motor neuron diseases; hereditary ataxias; hereditary motor sensory neuropathies (HMSN); hereditary paraplegias; fibromyalgia; amyotrophic lateral sclerosis (ALS); myasthenia gravis; and Pompe disease. The muscle disorder is preferably a congenital muscle disease or congenital muscular dystrophy.

As used herein, the term "subject" encompasses humans and animals. Preferably, a subject is a mammal, more preferably a human.

The term "therapeutically effective amount," as used herein, refers to an amount of muscle tissue being administered sufficient to relieve one or more of the symptoms of the disease or condition being treated to some extent. This can be a reduction or alleviation of symptoms, reduction or alleviation of causes of the disease or condition or any other desired therapeutic effect. Preferably, therapeutically effective amount is an amount sufficient to restore or establish normal functioning of skeletal muscle tissue that is replaced or regenerated using the muscle tissue of the invention.

BRIEF DESCRIPTION OF DRAWINGS

These and other features, aspects, and advantages of the apparatus, systems and methods of the invention will become better understood from the following description, appended claims, and accompanying drawing wherein:

FIG 1 illustrates fabrication methods of 3D culture chambers for tissue engineered skeletal muscles;
FIG 2 illustrates generation of 3D culture chambers;
FIG 3 illustrates Tissue Engineered Skeletal Muscles (TESMs) of different sizes generated with different 3D culture chambers;
FIG 4 illustrates an overview on the different fabrication methods described herein;
FIG 5 illustrates technical drawings showing the geometrical features and dimensions of the three different devices;
FIG 6 illustrates a program interface for printing a master mold;
FIG 7 provides an illustration of the "Direct Peeling method";
FIG 8 provides an illustration of the "ESCARGOT method";
FIG 9 provides an illustration of the "Ecoflex Replica method";
FIG 10 illustrates a 3D design;
FIG 11 illustrates a comparison between 3D printed positive and PDMS replicas produced with the Ecoflex Replica method;
FIG 12 illustrates bright field microscopy details of original master and PDMS replica made with the Ecoflex Replica method;
FIG 13 illustrates Scanning Electron Microscopy pictures;
FIG 14 illustrates bright field micrographs;
FIG 15 illustrates spontaneous compaction of TESMs in different chambers;
FIG 16 illustrates optimization generating muscle in a muscle-on-chip device;
FIG 17 illustrates effect of fibrin concentration on contractile force of in vitro cultured 3D skeletal muscle.
FIG 18 illustrates functional analysis of tissue engineered skeletal muscle cells (TESMs).
FIG 19 illustrates Pax7-positive muscle stem cells formed by TEMs.

DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise, it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

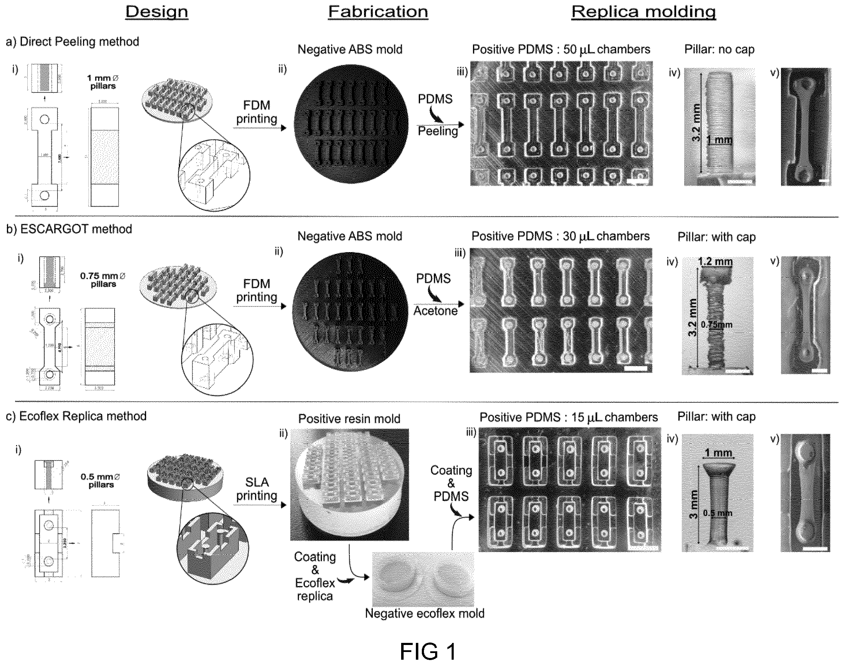

FIG 1 illustrates fabrication methods of 3D culture chambers for tissue engineered skeletal muscles. The present figure provides an overview of three different methods which are dubbed the "Direct Peeling method", the "ESCARGOT method", and the "Ecoflex Replica method". It will be understood that where reference is made herein to the "Ecoflex Replica Method", this method is preferably applied using Ecoflex or similar elastomeric materials, but of course can also be applied using other stretchable and moldable materials, (a) Direct Peeling method. Technical drawing of a single chamber and 3D CAD model of the negative mold (i), 3D printed acrylonitrile butadiene styrene ("ABS") negative mold through Fused Deposition Modelling ("FDM") printing (ii), detail of a polydimethylsiloxane ("PDMS") positive replica (iii), detail of the pillar (iv). (b) ESCARGOT method. The same process of the Direct Peeling method from design to 3D printed mold is shared (i-ii), detail of PDMS replica (iii) and of a T-shaped pillar with cap, obtained after the dissolution of the ABS mold (iv). (c) Ecoflex Replica method. Technical drawing of a single chamber and 3D CAD model of the positive mold (i), 3D printed resin positive mold through Stereolithography (SLA) printing (top), negative molds in form of replicas made of Ecoflex (bottom), detail of a PDMS positive replica (iii) and a T-shaped pillar provided with conical cap (iv). (a-c) Examples of tissue engineered skeletal muscles within the three different 3D chambers (v). Scale bars: **a-c,** 5 mm (iii), 1 mm (iv, v). 3D models of each design were generated using CAD software. For the FDM printed devices, 3D models were converted and then uploaded to the 3D printer. For the SLA printed platforms, 3D models were sliced.

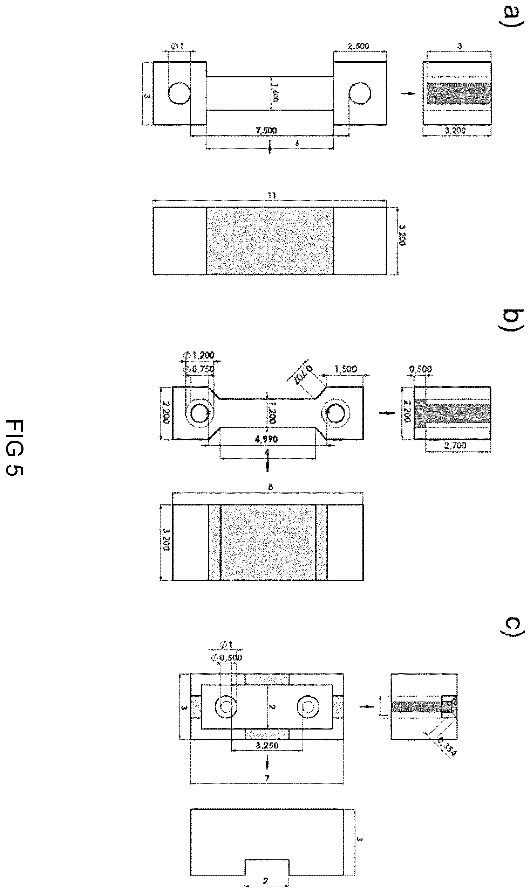

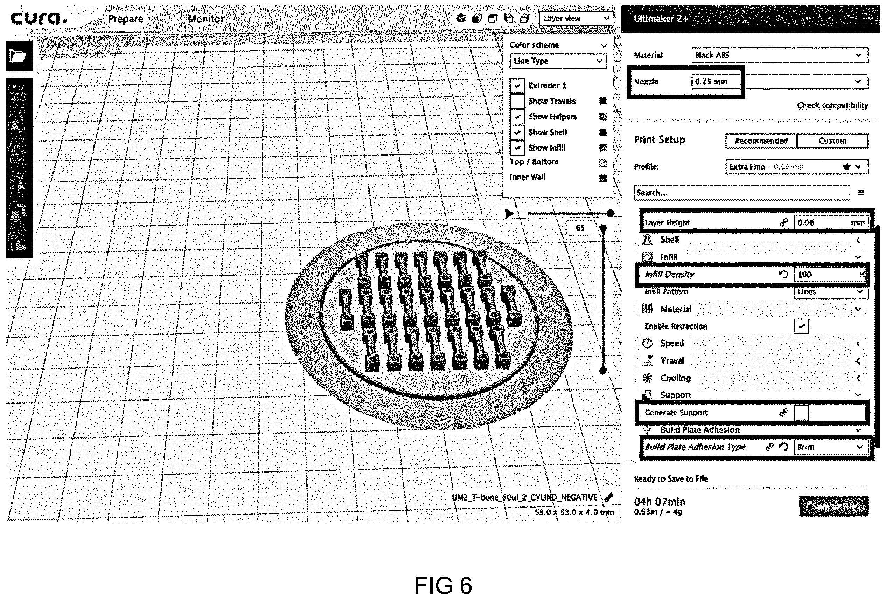

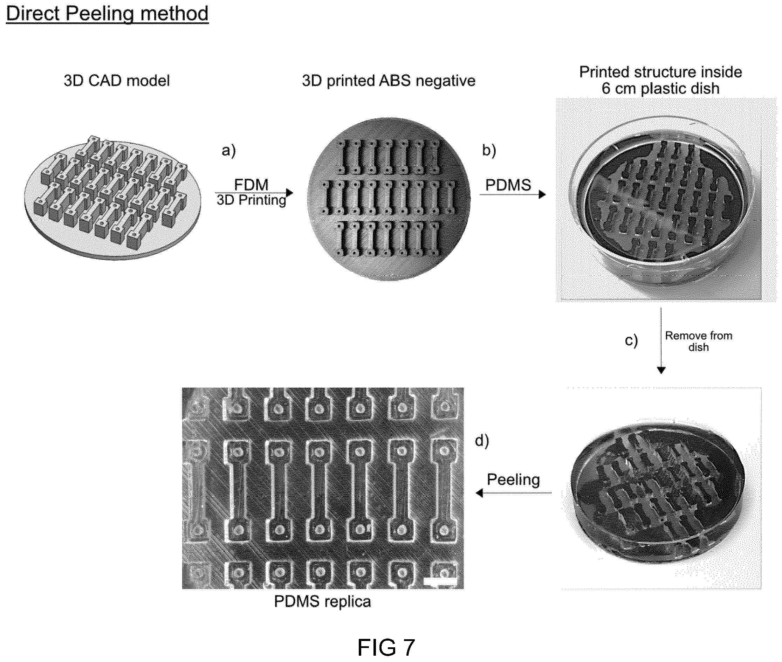

As illustration of the "Direct Peeling method" a first design is used including a 3D culture chamber of 50 μL in a "bone" shape with a total length of 11 mm, provided with two cylindrical pillars of 1 mm in diameter and 3.2 mm in height (**FIG 1a,** i; **FIG 5a**). This model was designed without overhanging feature of micrometric size, in order to be directly peeled from the 3D printed negative mold during the replica molding without incurring any damage to the final PDMS structure. A circular-based negative structure provided with a set of 23 chambers (**FIG 1a, ii**), was printed using an FDM 3D printer ("Ultimaker 2+") equipped with a 250 μm nozzle (**FIG 6**). The circular shape of the base was chosen to fix the printed mold inside a 60 mm plastic Petri dish for convenient PDMS casting during the replica molding step (**FIG 7**). For this method, e.g. polylactic acid (PLA) or acrylonitrile butadiene styrene (ABS) can be used as thermoplastic materials in 3D printing. ABS is preferred e.g. because PLA has a lower melting point compared to ABS and it can deform during the curing of the PDMS (>50°C). This method can have advantages of being relatively fast and user-friendly. However, the method can also be limited by the difficulty of obtaining replicas with more complex geometries, e.g. due to the mechanical damage that the peeling from a rigid mold can cause. For example, the hard, non-deformable mold makes the retention of features such as long and thin or T-shaped pillars arduous. This obstacle has led the inventors to development of the next two methods.

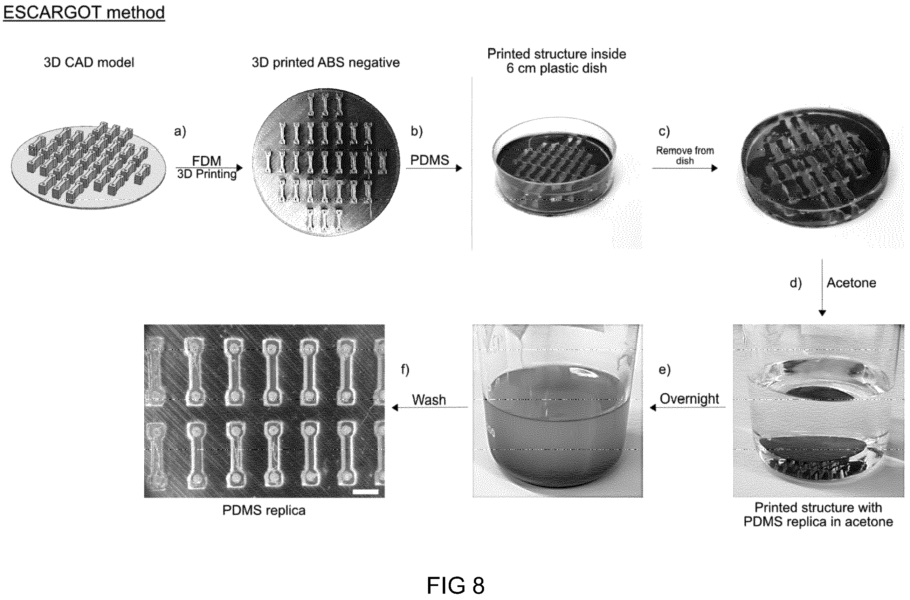

As illustration of the "ESCARGOT method", another design is used. Increasing the number of samples for experimental necessities often means reducing the volume of the sample itself (*e.g.* from a 12 wells plate to a 48 wells plate). However, reducing the diameter of a pillar can cause a contractile tissue to slip away from it due to the tension to which the bundle is subjected during culture. Hence, it is preferred to incorporate a protrusion in the pillar's design arises, leading to the more complex T-shaped pillar suitable for supporting small tissues. To this end, a negative model is used comprising a smaller, 8 mm long chamber of 30 μL with pillars of 750 μm in diameter and 3.2 mm in height in a T-shape (**FIG 1b** i; **FIG 5b**). To obtain a PDMS replica of such structure while still using FDM printing, we adapted another approach previously used in the fabrication of microfluidic systems in a monolithic PDMS [Saggiomo et al. "Simple 3D Printed Scaffold-Removal Method for the Fabrication of Intricate Microfluidic Devices", Advanced Science 2, 1500125 (2015)]. This method, called Embedded Scaffold Removing Open Technology (ESCARGOT), replaces the peeling with the dissolution of the mold by exploiting the solubility of ABS in acetone. Briefly, an ABS FDM-printed negative mold (**FIG 1b, ii**) was immersed in liquid PDMS, leaving at least one portion of the structure exposed. Once the PDMS was cured, the ABS scaffold was dissolved in an acetone bath overnight (**FIG 8**). The resulting PDMS positive structure retains all the features of the original model (**FIG 1b,** iii, iv), which would have been lost if peeled from the 3D printed mold. This method was developed to solve problems related to the need of smaller and more complex shapes of pillars, while still making use of the same 3D printing setup. However, for reproducible fabrication of pillars with a

diameter of 750 $\mu$m, or less, a further improvement is desired.

As illustration of the "Ecoflex Replica method", another design is used. The generation of 3D culture chambers with 15 $\mu$L volumetric capacity, containing T-shaped pillars with a diameter of 500 $\mu$m and a height of 3 mm (**FIG 5c**) was made possible employing a new method, which combines stereolithography (SLA) printing and fully deformable elastic molds (**FIG 1c**). SLA printers use a laser source to photo-crosslink a liquid polymeric resin, usually composed of acrylates, layer by layer. Thanks to the smaller laser spot, they are not only able to generate smaller features and thinner layers than FDM (down to 200 and 25 $\mu$m, respectively), but also to create complex geometries with overhanging structures. In some embodiments, a category of printers called masked SLA (mSLA) is used. For example, in these machines an array of 405 nm UV-LED can be used to replace the laser source.

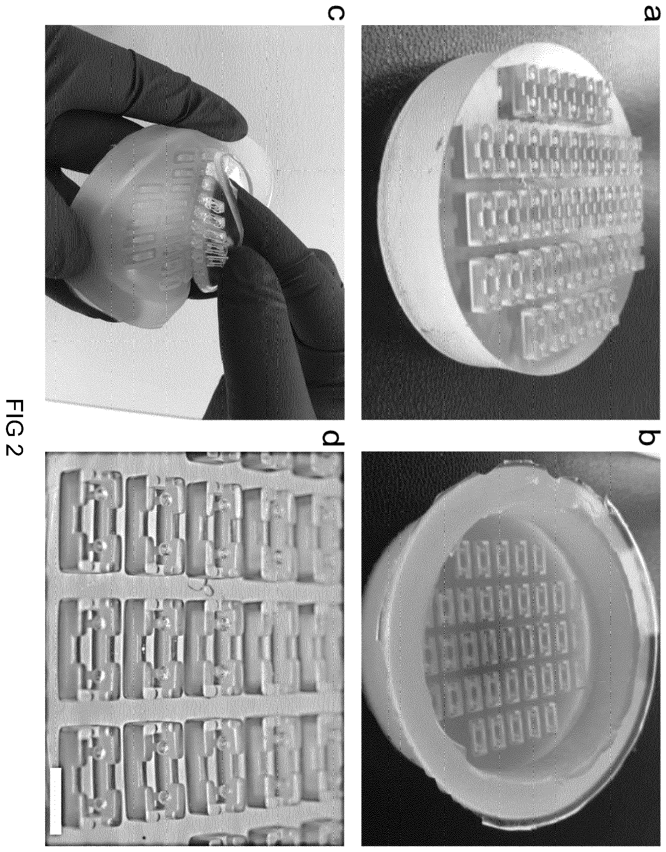

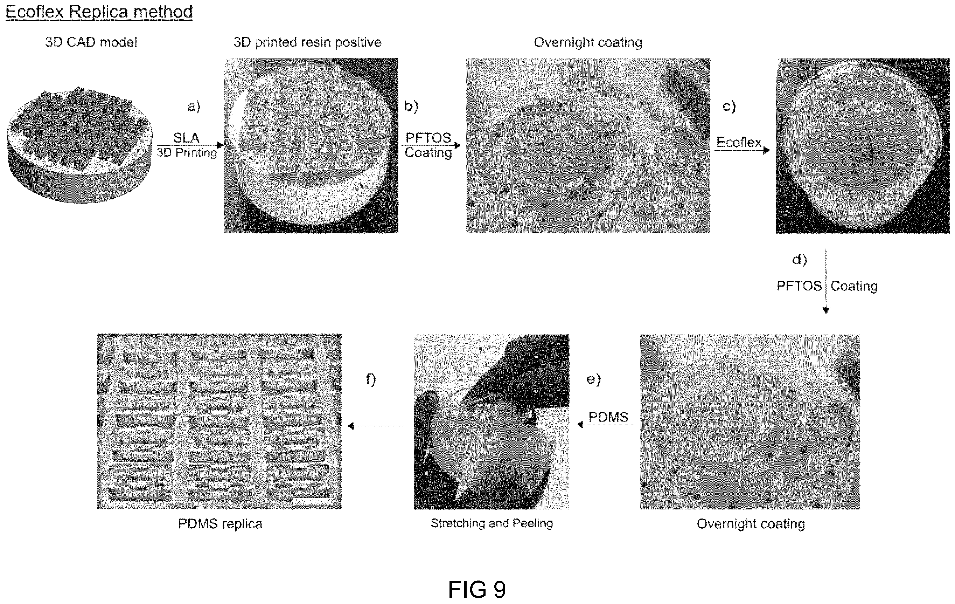

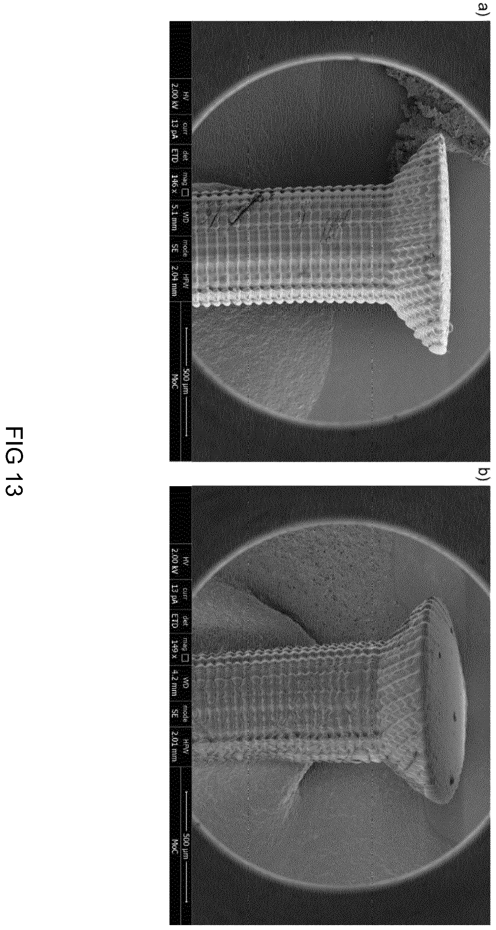

Despite the improved resolution of SLA printers, it can still be difficult to directly peel the PDMS replicas from the 3D-printed molds without damaging the micrometric features. In a preferred embodiment, this is addressed by generating an intermediate "carrier" mold made of the highly elastomer, most preferably comprising or essentially consisting of polybutylene adipate terephthalate (PBAT). For example, Ecoflex 00-30 can be used which is a cheap, stretchable and durable silicone, mostly used in soft robotics. According to the datasheet Ecoflex™ 00-30 has Mixed Viscosity (ASTM D-2393) 3,000 cps; Specific Gravity (ASTM D-1475) 1.07 [g/cc], Specific Volume (ASTM D-1475) 26.0 [cu. in./lb.], Pot Life (ASTM D-2471) 45 min.; Cure Time 4 hours; Shore Hardness (ASTM D-2240) 00-30; Tensile Strength (ASTM D-412) 200 psi; 100% Modulus (ASTM D-412)10 psi; Elongation at Break %(ASTM D-412) 900%; Die B Tear Strength (ASTM D-624)38 pli; Shrinkage (ASTM D-2566)< .001 in./in. So for example, the material can be stretched over 900 percent before breakage. In some embodiments, e.g. as shown, Ecoflex or similar material is used as negative mold to form PDMS replicas. In one embodiment, e.g. as shown, we used an SLA 3D printer, e.g. Formlab Form 2 SLA or a Photon Anycubic mSLA, to 3D print a positive structure comprising 37 culture chambers of 7x3x3 mm (LxHxW) equipped with 3 mm long T-shaped pillars of 500 $\mu$m diameter, provided with conical caps (**FIG 1c**, i, ii; **FIG 2a**; **FIGs 12**, **13**). The negative mold was then generated through Ecoflex Replica molding (**FIG 1c**, ii, iii; **FIG 2b**) and the final positive PDMS was easily peeled off after stretching the Ecoflex mold (**FIG 1**, iii; **FIG 2c**, **d**). In a preferred embodiment, both the 3D printed positive and the Ecoflex negative molds were subjected to a perfluorodecyltrichlorosilane (PFOTS) coating, which facilitates the unmolding process by making their respective surfaces inert (see **FIG 9**). Being subjected to reiterated mechanical and thermal stresses, Ecoflex intermediate molds appear to acquire structural wear over time. Nevertheless, we found that a single Ecoflex mold with 37 chambers could be re-used at least 15 times without issues, yielding over 500 3D chambers with intact T-shaped pillars and showing only micro-cracking (**FIG 13**).

**FIG 2** illustrates generation of 3D culture chambers through the Ecoflex Replica method. (**a**) Positive mold in acrylate resin produced with SLA 3D printing. (**b**) Single negative replica obtained by pouring liquid Ecoflex on the 3D printed positive from **a**, after curing of the elastomer. (**c**) Ecoflex Replica molding: PDMS was poured inside the Ecoflex negative mold. After curing the PDMS, the mold was stretched, allowing easy peeling of the PDMS positive replica, (**d**) Detail of the PDMS positive replica: the micrometric features of the chambers and the caps on the pillars are retained. Scale bar: **d**, 5 mm.

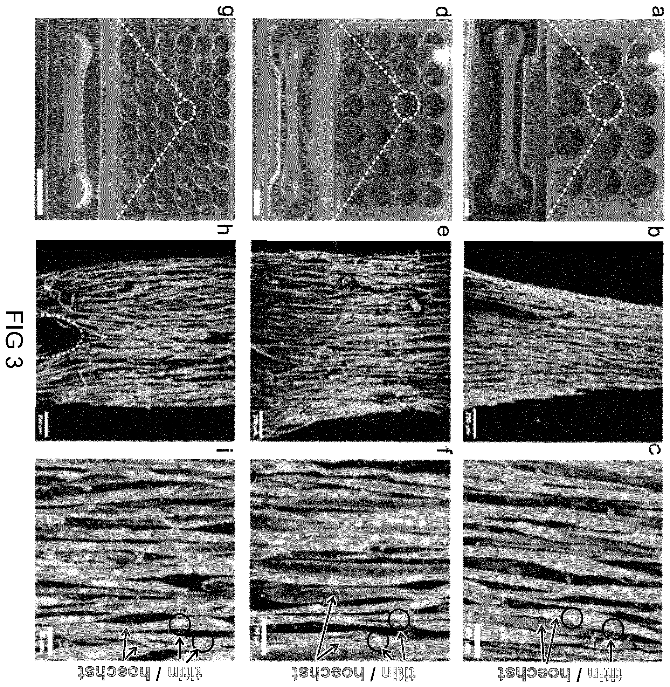

**FIG 3** illustrates Tissue Engineered Skeletal Muscles (TESMs) of different sizes generated with the three different 3D culture chambers. (**a-c**) 12 well plate containing TESMs within the 50 $\mu$L chambers obtained with the Direct Peeling method (**a**). Tissues were stained for the sarcomeric protein titin after 7 days of differentiation (**b**); the typical sarcomeric striation pattern of titin is visible at higher magnifications (**c**). (**d-f**) Smaller, 30 $\mu$L TESMs, obtained from the ESCARGOT fabricated chambers, inside a 24 well plate (**d**); engineered tissues display a similar morphology as in **b** with millimetres-long myotubes (**e**) with evident sarcomeric striation (**f**). (**g-i**) A 48 well plate including TESMs generated in the 15 $\mu$L chambers fabricated with the Ecoflex Replica method (**g**). Even in the smallest tissue, myogenic progenitors differentiated in long, multinucleated myotubes with an organized titin pattern (**h**, **i**). The dashed curved line at one extremity of the TESM indicates a loop-like structure originated from the tension to which the tissue is subjected (g, h). Scale bars: **a**, **d**, **g**, 1 mm; **b**, **e**, **h**, 200 $\mu$m; c, **f**, **i**, 50 $\mu$m.

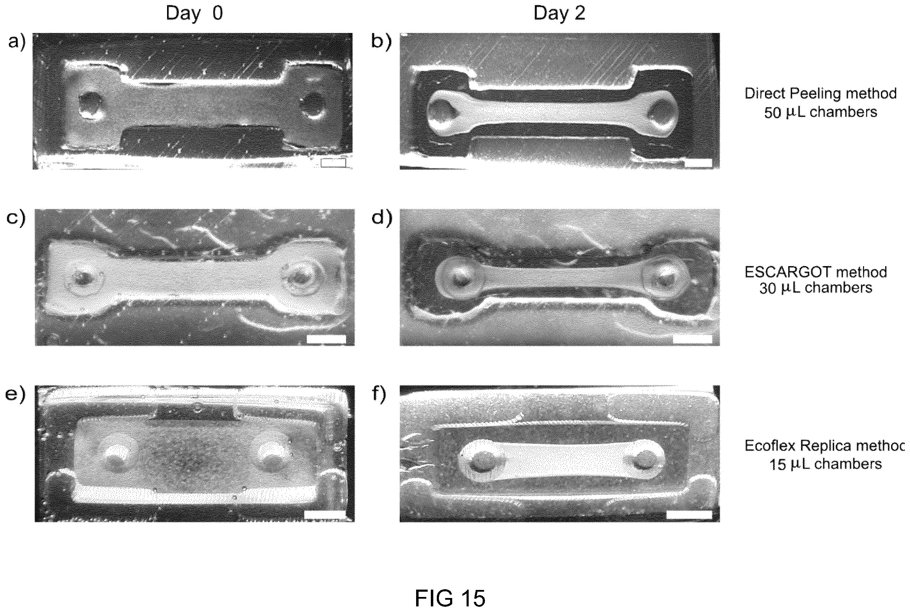

Some aspects of the invention may relate to specific uses of the microscopic structures as described herein. As an example, we discuss generation of Tissue Engineered Skeletal Muscles (TESMs). We determined the generated 3D tissue chambers were able to promote the formation of TESMs and how downscaling affects formation of 3D tissues. TESMs were generated by mixing a suspension of human iPSC-derived myogenic progenitors, with a biocompatible hydrogel constituted of fibrin and Matrigel (see Methods) developed by adapting a previously published protocol [Hinds et al., "The role of extracellular matrix composition in structure and function of bioengineered skeletal muscle", Biomaterials 32, 3575-3583 (2011)]. Corresponding volumes of 50 $\mu$L, 30 $\mu$L and 15 $\mu$L of cell-laden hydrogel were directly pipetted in the chambers obtained from the Direct Peeling method, ESCARGOT method and Ecoflex Replica method, which were fixed inside the wells of a 12 well plate, 24 well plate and 48 well plate, respectively (**FIG 3**). As an example, from an initial cell-hydrogel volume of 500 $\mu$L it was possible to cast 10 units of 50 $\mu$L TESMs, 16 units of 30 $\mu$L TESMs, while from the same volume 33 TESMs could be casted in the devices obtained with the Ecoflex Replica method. In the first two days after casting, the cell-laden hydrogel underwent visible compaction (**FIG 15**). After 7 days of differentiation, TESMs were fixed, immunostained and analysed by confocal imaging (see Methods). Engineered tissues in all the 3D culture systems showed a comparable organization, characterized by the presence of long, aligned and multinucleated myotubes that stained positive for the sarcomeric protein titin (**FIG 3b**, e, h), whose typical striated pattern was immediately recognizable (**FIG 3c**, f, i).

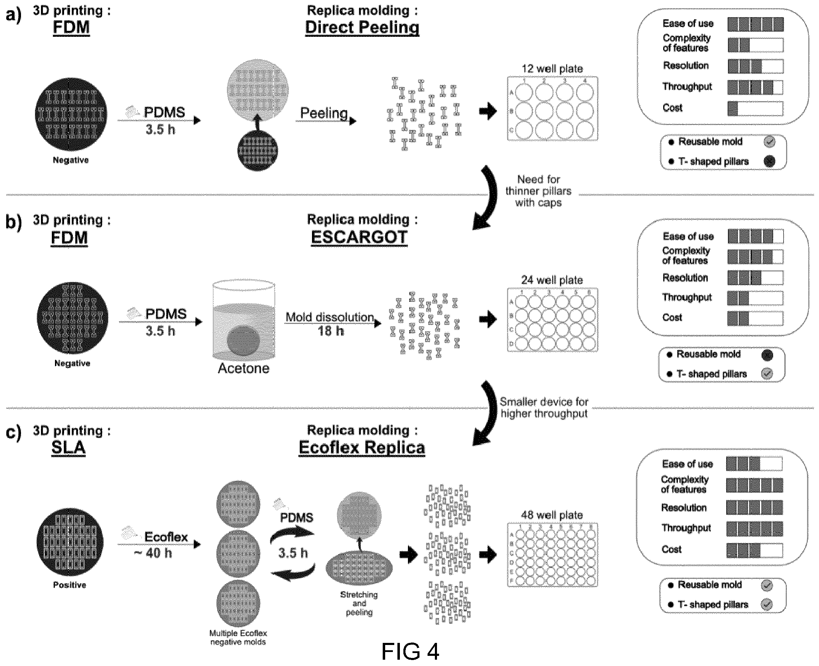

**FIG 4** illustrates an overview on the different fabrication methods described herein. (**a**) The Direct Peeling method is the fastest and easiest to the operator, involving a simple design and a reusable negative mold, thus maintaining a noticeable low cost. (**b**) With the same FDM setup, in the second method it is possible to generate thinner pillars provided with caps still in a single demolding step. In this case the 3D printed mold is dissolved in acetone, therefore not reusable, with a slight increase in production cost. (**c**) For higher throughput applications, the Ecoflex Replica method allows both an increment of resolution and production, thanks to the use of the SLA printer and the stretchable and reusable negative molds. (**a-c**). All the three methods can be operated in any standard biomedical laboratory without the need of specific biofabrication expertise. Ease of use indicates the number of steps and equipment required to obtain a certain device; complexity of features refers to the multiplicity of features that can be simultaneously incorporated in the final product; resolution indicates the capacity of the 3D printer to print small details; throughput indicates the number of devices that can be produced per unit of time and their suitability for standard multi-well systems; cost refers to the initial investment of equipment, while the cost of the fabrication materials are comparably low for each method.

The invention illustrates a fabrication pipeline which can be based, e.g., on off the shelf 3D printers, comprising three methods to generate PDMS-based tissue engineering culture devices. We have highlighted the adaptability of our approach by producing 3D culture chambers to support TESMs with different sizes and levels of complexity (**FIG 4**). In principle, the structures realized with the Direct Peeling method can be produced with other fabrication techniques, such as milling or injection molding, thanks to their millimetric dimensions. However, 3D printing offers higher versatility, as the fabrication process can be fully tuned in-house from design to manufacturing to fit different needs and possible new necessities naturally arising during research. An example of this versatility is represented by the second method, developed using the same FDM printing setup but implementing the ESCARGOT strategy. To meet the need of thinner, T-shaped pillars provided with "caps" for a better retention of smaller engineered muscles, the ABS plastic molds were dissolved in acetone leaving the features intact.

In the Ecoflex Replica method, an SLA 3D printer was employed to further downscale the system with increased reproducibility for higher throughput applications. The devices produced with SLA 3D printing include the simultaneous presence of millimetric and micrometric features, a combination that is hardly possible with techniques such as photolithography, which is confined to the realization of structures within hundreds of micrometers. To replicate such structures in PDMS, a highly elastomer such as Ecoflex can used as a re-usable replica molding substrate for tissue engineering applications. To avoid accidental damage during demolding, other procedures may require supplementary steps solely to fix caps on each pillar or serial replica molding stages with high risk of failure. We inexpensively produced hundreds of faithful replicas with T-shaped pillars without additional fabrication steps thanks to the implementation of the stretchable molds. Considering the advantages, the low cost and the simplicity of use, the Ecoflex Replica strategy can be employed in a wide spectrum of applications: from the creation of supporting devices for other contractile or load bearing tissues such as heart and tendons, to the direct use as soft substrate for specific cell culture needs. The versatility, speed, low cost and ease of use of our methods can promote a larger diffusion of tissue engineering approaches in biomedical laboratories, and their implementation as tools for basic research, disease modeling and drug screening.

In the following we discuss specific devices materials, and methods which were used to produce some of the present results. Of course, it will be understood that the invention is not limited to these specifics. For example, where specific printing processes are described, also other similar processes can be used. For example, where reference is made to Ecoflex as the preferred material, also other elastomers can be used, e.g. allowing to be stretched before breaking by at least a factor two, three, five, ten, or more. For example, where reference is made to PDMS also other (viscoelastic) elastomers can be used.

In some embodiments, FDM printing is performed using a 3D printer, equipped with Acrylonitrile Butadiene Styrene (ABS) plastic. For example, the nozzle has 0.250 mm diameter, using a layer thickness of 0.060 mm and a controlled extruding temperature of 240°C. For the present results, SLA printing was performed both by means of a Form2 (Formlabs, USA) using grey resin v4 (Formlabs, USA) and a layer thickness of 0.050 mm and of an Anycubic Photon using Anycubic 405 UV clear resin (Anycubic, China) and a layer thickness of 0.050 mm. In one embodiment, after printing, the SLA products are left in isopropanol. In another or further embodiment, the SLA products are left in ethanol (EtOH). For example, the products are left in isopropanol and ethanol for twenty and ten minutes respectively. In some embodiments, the products are finally rinsed, e.g. with EtOH. In one embodiment, the prints are dried, e.g. with compressed air. In another or further embodiment, the prints are post-cured, e.g. using UV light and/or heat. For example, the prints are left in an 80W UV chamber for 10 minutes. In some embodiments, the 3D printed molds are coated. For example, the molds are coated with trichloro(1H, 1H, 2H, 2H-perfluorooctyl)silane (e.g. PFOTS, 97%, Merk). In one embodiment, the coating is applied using chemical vapour deposition (CVD), e.g. in a vacuum desiccator. For example, the 3D printed structure is first air plasma activated (e.g. for 30 seconds), then placed in a desiccator with a vial of PFOTS (e.g. 100 $\mu$L) and high vacuum is applied. In some embodiments, the desiccator is left under static vacuum for some time for CVD, e.g. for several hours. After PFOTS deposition, the 3D printed structure can be removed from the desiccator. In one embodiment, the structure is left in an oven, e.g. at 70°C for 1 hour and finally washed with EtOH and isopropanol.

In some embodiments, Ecoflex 00-30 (e.g. Smooth-On Inc., PA) intermediate mold is generated by pouring the two pre-mixed liquid pre-polymers (1A:1B) on the 3D printed structure. This can be followed by a degassing step, e.g. inside a vacuum desiccator for 15 minutes. After degassing, platforms can be left curing, e.g. at room temperature (RT) for 8 hours. In one embodiment, the platforms can be peeled off the 3D printed original mold. In some embodiments, the Ecoflex mold is washed, e.g. with EtOH, and rinsed dry, e.g. with compressed air. In a preferred embodiment, before being utilized as negative molds for PDMS replica molding, Ecoflex molds can be subjected to a coating, e.g. PFOTS CVD coating using the same procedure described for the CVD of the 3D printed mold.

In some embodiments, Polydimethylsiloxane (PDMS, e.g. Dow Corning, MI) final chambers are produced by pouring the uncured elastomer. For example, the uncured elastomer is previously mixed in a 10:1 w/w ratio of pre-polymer and curing agent and degassed for 10 minutes, on the ABS or Ecoflex molds. In some embodiments, poured PDMS is again degassed for 20 minutes in order to remove any possible trapped air bubble and subsequently cured, e.g. at 75°C in a ventilated oven for at least 3 hours. In other or further embodiments, cured PDMS replicas are peeled off the molds. Preferably, the replicas are rinsed, e.g. in isopropanol, to remove any impurities.

In some embodiments, ABS dissolution is achieved by immersing the 3D printed negative structure for a few hours, e.g. overnight, in an acetone bath, together with its PDMS positive still attached. Complete removal of ABS can be helped by washing the PDMS replica under running acetone flow for few seconds.

In some embodiments, in order to be utilized for 3D cell culture, each 3D chamber is cut from the whole PDMS block. In one embodiment, the chamber is fixed inside wells of e.g. 12, 24 or 48 well plates (e.g. CELLSTAR®, Greiner Bio-One, Germany) using PDMS (10:1) as glue and allowing it to solidify for 1 hour at 70°C. Preferably, the PDMS replicas from each fabrication method are sterilized, e.g. by rinsing in 70% ethanol for 15 minutes followed by 3x PBS washing and by UV treatment for 15 minutes, immediately before usage in cell culture.

In some embodiments, human iPSC-derived myogenic progenitors are generated following a previously published protocol [Saggiomo et al., "Simple 3D Printed Scaffold-Removal Method for the Fabrication of Intricate Microfluidic Devices", Advanced Science 2, 1500125 (2015).] and cultured accordingly. For example, cells are expanded in monolayer culture in myogenic progenitor proliferation medium, consisting of DMEM high glucose (e.g. Gibco, Waltham MA) supplemented with 10% FBS (e.g. Hyclone, US), 1% penicillin-streptomycin-glutamine (P/S/G) (e.g. Gibco, Waltham, MA) and 100 ng/mL bFGF2 (e.g. Prepotech, Rocky Hill, NJ); medium was refreshed, e.g. every 48 hours. Culture dishes of 100 mm (e.g. CELLSTAR®, Greiner Bio-One, Germany) were coated with ECM extract (1:200 diluted, e.g. Sigma-Aldrich, E6909) 40 minutes prior seeding. For passaging and harvesting, cells were detached in the incubator at 37°C and 5% $CO_2$ with TrypLE reagent (e.g. Gibco, Waltham, MA) diluted 1:1 with PBS (e.g. Gibco, Waltham, MA).

In some embodiments, hydrogel generated as extracellular matrix for the 3D culture is composed of bovine fibrinogen (e.g. Sigma-Aldrich) dissolved in DMEM high glucose (final concentration: 2 mg/mL), Matrigel growth factor reduced (20% v/v, e.g. Corning Life Sciences, Netherlands), thrombin from human plasma (e.g. Sigma-Aldrich) dissolved in 0.1 % BSA in PBS (1% v/v, 0.5 U/mL final concentration), myogenic progenitor proliferation medium (69% v/v). Matrigel, previously stored at -20°C, was kept at 4°C 2 hours before usage; fibrinogen, stored at -80°C, was kept at 4°C 1 hour before usage. Myogenic progenitors used for the generation of the TESMs were harvested before passage 10. After detachment from culture dishes, cells were suspended in myogenic progenitor proliferation medium at a concentration of $16 \cdot 10^6$ cells/mL and the suspension was then mixed with fibrinogen and Matrigel. Lastly, thrombin was added to the mix immediately before pipetting the cell-hydrogel mix into the 3D chambers, which were coated with Pluronic F-127 (e.g. Sigma-Aldrich) for 1 hour at RT to prevent unwanted adhesion of the hydrogel. A volume of 50 μL, 30 μL and 15 μL was used for each TESM in the chambers obtained with the Direct Peeling, ESCARGOT and Ecoflex Replica methods, respectively. All the hydrogel components, as well as tubes and micropipette tips were kept on ice prior and during the duration of the experiments. The final solution was then left to polymerize for 30 minutes in the incubator at 37°C and 5% $CO_2$, before adding the myogenic progenitors proliferation medium supplemented with 6-aminocaproic acid (1.5 mg/mL, e.g. Sigma-Aldrich). After 48 hours, proliferation medium was switched to TESM differentiation medium, consisting of DMEM high glucose supplemented with 1% knock-out serum replacement, 1% ITS-X (all Gibco), 1% penicillin-G (e.g. Sigma-Aldrich), 1% Glutamax (e.g. Sigma-Aldrich), 6-aminocaproic acid (1.5 mg/mL, e.g. Sigma-Aldrich). Half volume of the TESM differentiation medium was replaced every 48 hours. TESMs were kept on agitation at 55 rpm (e.g. Celltron orbital shaker, Infors HT, Switzerland) at 37 °C and 5% $CO_2$.

In some embodiments, Immunofluorescence Stainings are applied as follows. Samples were fixed in 4% paraformaldehyde (PFA, e.g. Sigma-Aldrich) for 1 hour at RT and then washed with PBS for three times. For whole-mount immunostaining, fixed samples were first subjected to a permeabilization/blocking step in a solution containing 0.5% Triton-X in PBS, 3% BSA in PBS, 0.1% Tween 20 in PBS on agitation, for 1 hour at RT. After washing with PBS, samples were incubated with primary antibody for titin, 9D 10-s (DSHB, IA), in 0.1% Triton-X in PBS, 0.1% BSA (e.g. Sigma-Aldrich) in PBS, 0.1% Tween 20 in PBS at 4°C, for 1 hour. Before incubation with the secondary antibody Alexa Fluor 488 (Thermo Fisher Scientific, Waltham, MA), samples were washed in 0.1% Tween in PBS for 2 minutes and subsequently in PBS for 2 minutes. Secondary antibodies were diluted in the same solution used for the primary antibodies and samples were incubated for 1 hour at RT. Lastly, nuclear staining was performed through incubation with Hoechst 33342 (1:15000, e.g. Sigma-Aldrich) at RT for 15 minutes followed by washing in PBS. Samples were kept in PBS at 4 °C before imaging. In some embodiments, stained samples were imaged using a Leica TCS SP5 confocal microscope (Leica, Germany) equipped with LAS software (Leica, Germany), using 10x and 20x magnifications.

With reference to the "Direct Peeling" and "ESCARGOT" methods, the fabrication steps can be largely identical. However, different demolding steps can be used in order to obtain PDMS replicas from the different masters.

Fused Deposition Modeling (FDM) 3D printers are the most commonly used 3D printers. In short, a heated nozzle deposits a thermoplastic material layer by layer on a build plate. FDM printers are relatively simple to use and to maintain. Typically, FDM printers have a standard 400 $\mu$m nozzle. However, the most suitable option for structures that need more precision is the 250 $\mu$m nozzle. Although it is possible to print ABS with many printers, it is preferred to use a printer with an enclosure as the ABS can warp due to different temperatures experienced by the 3D printed material on the build plate.

Various programs can be used to design the 3D masters, depending on the difficulty of the program, operating system and personal preferences. Once the design is completed, the 3D design is typically saved as .STL file.

For 3D printing, the STL file is typically "sliced" in layers. This can be done with slicer programs. In the slicer program, many variables can be changed. For example, the nozzle size can be set to 250 $\mu$m, the layer height to 60 $\mu$m, 100% infill and print with the use of brim as build plate adhesion but without support (**FIG 6**). Other variables such as extrusion temperature, bed temperature, speed and fan speed can also be changed according to the material to be printed. Once the slicer has finished, the file can be saved, e.g., as .gcode, and loaded on the 3D printer. Once the printer has finished, the 3D print can be easily detached from the build plate once it has cooled down to room temperature.

**FIG 5** illustrates technical drawings showing the geometrical features and dimensions of the three different devices. (a) 50 $\mu$L chamber produced with the Direct Peeling method, (**b**) 30 $\mu$L chamber obtained with the ESCARGOT method. (c) 15 $\mu$L chamber fabricated through the Ecoflex Replica method. The blue color highlights the pillar in each design.

**FIG 6** illustrates a program interface ("Cura") and variables to be changed for printing the master.

In the Direct Peeling method, the 3D printed (negative) master can be fixed, e.g., to the bottom of a 6 cm plastic petri dish by means of common double sided tape or a thin layer of PDMS (10:1). This will facilitate the subsequent removing of the structure. PDMS is mixed in a 10:1 PDMS/curing agent ratio for few minutes and degassed under vacuum for 10 minutes. The PDMS mixture can be then poured on top of the 3D printed negative master and degassed again for 15 minutes to remove any air bubble trapped inside. The PDMS is then cured in an oven at 75°C for 3 hours. Once the PDMS is cured, it can be peeled off from the negative master.

In the ESCARGOT method, instead of peeling off the PDMS from the negative mold, the structure comprised of the negative mold and the cured PDMS on top can be placed in an acetone bath overnight for dissolving the ABS. The acetone can be refreshed from time to time for speeding up the dissolution. Stirring and sonication can also speed up the dissolution process. After the mold dissolution, the remaining PDMS replica is quickly washed with running acetone and isopropanol, then air dried.

**FIG 7** provides an illustration of the Direct Peeling method. (**a**) The 3D CAD model is printed through FDM into an ABS negative mold. (**b**) The 3D printed mold is fixed inside a 6 cm plastic Petri dish with double sided tape; this will facilitate the subsequent pouring of uncured PDMS. (c) After curing of PDMS, the mold with PDMS still attached is removed from the dish. (**d**) The PDMS replica is carefully peeled off the negative mold; scale bar, 5 mm.

**FIG 8** provides an illustration of the ESCARGOT method, (**a**, **b**) The 3D CAD model is 3D printed into a negative mold and fixed inside a 6 cm plastic Petri dish, uncured PDMS is next poured in the dish and left to cure in the oven. (c, d) After curing of the elastomer and removal of the mold from the dish, the couple negative mold-PDMS replica is immersed in an acetone bath. (e) The negative mold will dissolve overnight, (f) The PDMS replica is finally washed with acetone; scale bar, 5 mm.

**FIG 9** provides an illustration of the Ecoflex Replica method. (a) The 3D CAD model is 3D printed through (m)SLA in a positive resin structure with a 1 cm thick base. (b) After a 30 seconds plasma activation step, the printed positive is coated with PFOTS overnight, (c) After treatment, a negative Ecoflex replica is obtained from the resin positive. The ecoflex replica is characterized by a 1 cm high wall which will facilitate pouring of PDMS inside the mold itself. (d) A second activation-coating step is performed on the Ecoflex negative, (e) Uncured PDMS is poured inside the mold, after curing it is easily peeled off by stretching the Ecoflex mold. (f) The final PDMS replica is obtained; scale bar, 5 mm.

SLA (stereolithography) printers, in contrast to FMD printers, use a liquid resin, typically made of (meth)acrylates mixtures, which is photocrosslinked, e.g. using specific wavelengths. Some printers use a 405 nm laser to photopolymerize the liquid resin layer by layer. Another class of SLA printers are called LCD (liquid crystal display) or mSLA (mask stereolithography) 3D printers. These machines can use a 405 nm LED arrays as light source and an LCD to create a mask for the crosslinking of the liquid resin layer by layer.

In some embodiments, the positive master is designed as follows. In the Ecoflex replica method the printed structure preferably serves as a template to create the Ecoflex negative mold. For this reason, the master is printed as a positive, such as the final PDMS replica which will be its copy. The software used to create the positive 3D model is however independent of the type of structure or the 3D printer used, and various 3D modelling programs can be used.

In some embodiments, printing and slicing of the 3D master is performed as follows. To print the model, the 3D model can be sliced. Preferably, the master is printed in a horizontal orientation, lying flat directly on the build plate surface without the use of support structures. Printing parameters may vary between different resin materials. Preferably, a layer height of around 50 $\mu$m is used. Once printed, the object can be cleaned, e.g. by leaving in an isopropanol bath for 20 minutes and then in an ethanol bath for an additional 10 minutes, to remove the uncured resin. The object can then be washed and dried e.g. using a flow of air. The 3D printed object is then post-cured in a UV chamber, e.g. for 10 minutes.

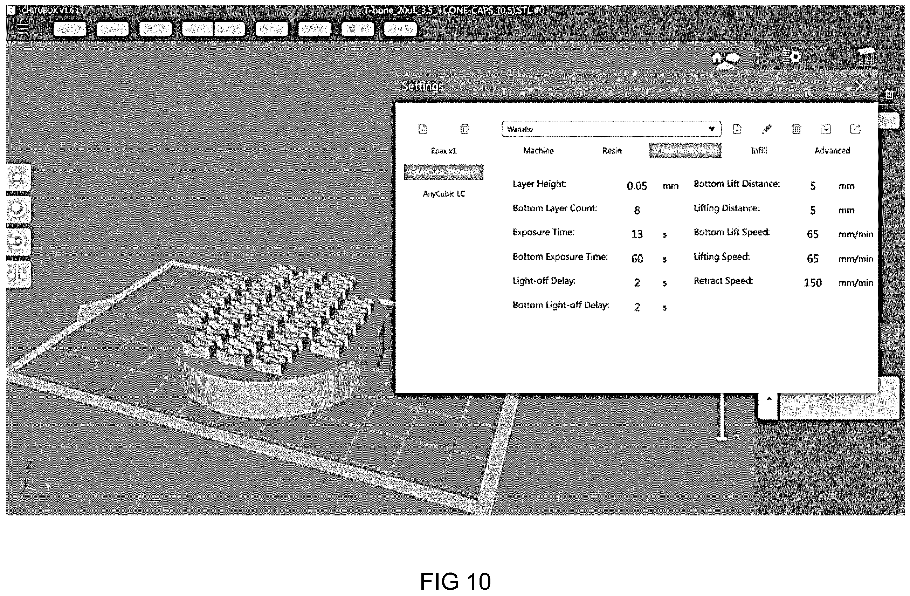

**FIG 10** illustrates a 3D design. The design is printed flat directly on the build plate without the use of supporting material. The parameters in the box are the ones used for printing on the Anycubic Photon using the Anycubic clear resin.

In some embodiments, coating of the master is performed as follows. Uncured (meth)acrylates may react with Ecoflex and PDMS, forming covalent bonds between the two surfaces. To eliminate this problem, a chemical coating can be used in some embodiments. For example, the 3D printed object is activated using an air plasma oven, e.g. for 30 seconds. A PE- 25 benchtop air plasma cleaner (e.g. Inseto Plasma Etch, Inc) was used at its maximum RF plasma power of 100 W with an air flow of ~ 10 cc/min, which allowed for a vacuum pressure of 200-250 mTorr within the chamber during plasma treatment. After the plasma treatment, the 3D printed object is placed in a 22 cm desiccator together with an open vial containing 100 $\mu$L of (1H, 1H, 2H, 2H-perfluorooctyl)silane (PFOTS). The desiccator is evacuated (2 x 10-3 mbar) and left under static vacuum overnight. After that, the 3D objects are left in an oven at 70°C for 1 hour and then washed with ethanol and isopropanol and dried with a flow of air.

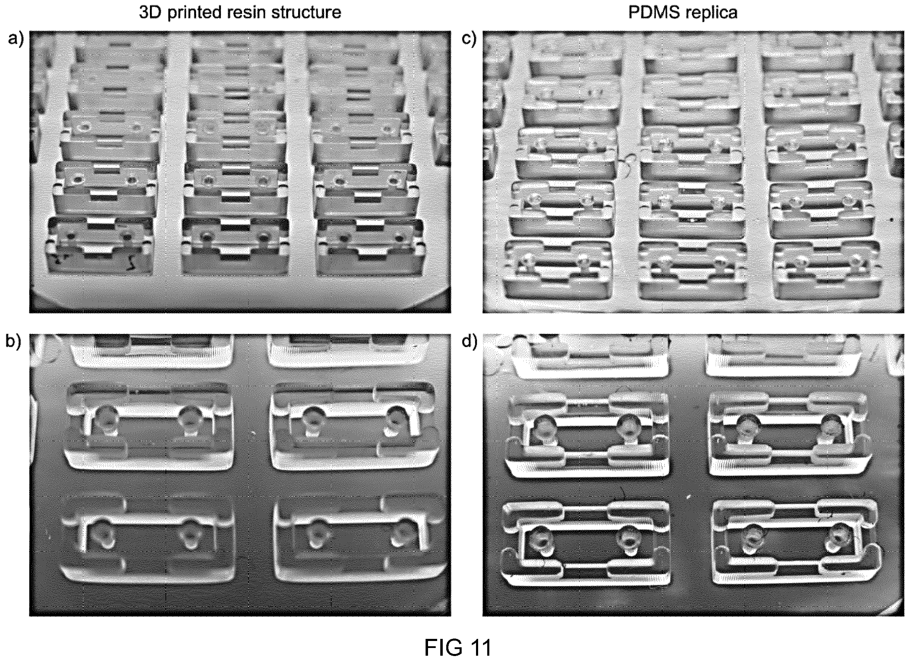

In some embodiments, Ecoflex Replica and demolding is performed as follows. The 3D positive mold is glued on a petri dish using hot glue. Ecoflex 00-30 is mixed 1:1 part A/part B and stirred for 5 minutes. The mix is then poured on top of the 3D positive mold and left under vacuum for 15 minutes. The Ecoflex was then left curing at room temperature for 8 hours. Once set, the Ecoflex negative mold was gently peeled from the 3D printed positive mold, washed with ethanol and dried using compressed air. Ecoflex, bearing the same silicone components of the PDMS can react with it forming covalent bonds. For this reason, the same coating procedure used in (2.4) was used to coat the Ecoflex negative mold. To generate the PDMS replica from the Ecoflex negative elastic mold, uncured PDMS is poured inside the mold and, after curing at 75°C for 3 hours, it is peeled off by stretching the Ecoflex. This procedure will allow the retention of the small and thin features of the PDMS replica. A visual comparison of the original 3D printed positive master and the PDMS positive replicas can be appreciated in FIGs 11-14.

**FIG 11** illustrates a comparison between 3D printed positive and PDMS replicas produced with the Ecoflex Replica method. (a,b) Original positive resin structure 3D printed with a SLA printer. (c, d) Final PDMS replica: the original general structure is retained after the demolding process.

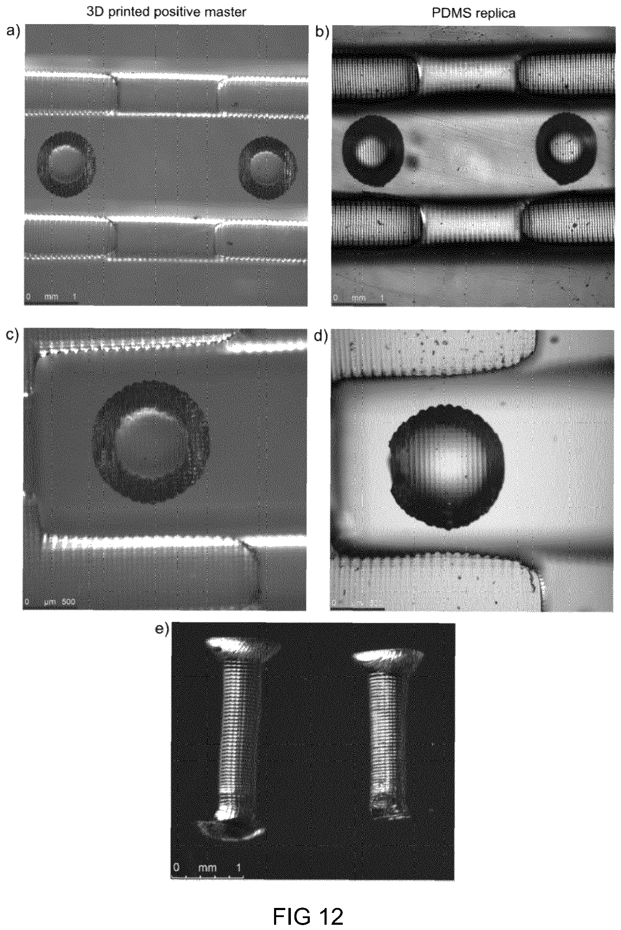

**FIG 12** illustrates bright field microscopy details of original master and PDMS replica made with the Ecoflex Replica method, (a) Original positive 3D printed single chamber. (b) PDMS replica of the same chamber. The micro-ridges of the original printed structure are retained in the PDMS replica, meaning that the intermediate Ecoflex negative mold can be used without losing resolution. (c) Detail of a pillar's cap. (d) PDMS replica of a pillar's cap. Even if a small defect is present, the general structure remains faithful to the original. (e) Side view of a 3D printed pillar (left) and its PDMS replica (right). Scale bars, a, b, e, 1 mm; c, d, 500 $\mu$m.

**FIG 13** illustrates Scanning Electron Microscopy pictures of an mSLA 3D printed pillar (a) and a PDMS replica (b). The SEM micrographs were acquired on a FEI Magellan 400 SEM. Scale bars, a, b, 500 $\mu$m.

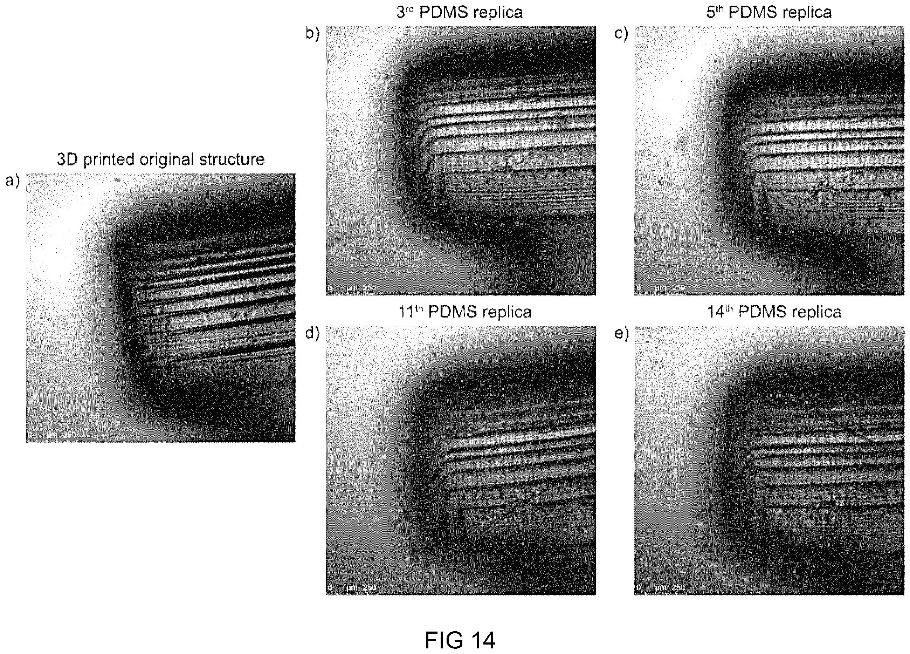

**FIG 14** illustrates bright field micrographs. Structural integrity of Ecoflex Replica products after several cycles of replica molding. (a) Detail of a 3D printed original resin structure fabricated by means of an SLA printer. (b) After 3 cycles of replica molding using the same Ecoflex negative mold, the PDMS replica starts to display signs of micro-defects, (c-e) Re-using the same Ecoflex mold after 14 cycles of heating and stretching doesn't impair the structural integrity of the features in the PDMS replica, while the micro-defects show only a slight expansion. Scale bars, a-e, 200 $\mu$m.

We will now discuss advantageous uses of the microstructures obtainable by the methods of manufacturing as described herein. One particular use pertains to the generation of the TESMs in 3D chambers with microstructures made of PDMS.

**FIG 15** illustrates spontaneous compaction of the TESMs in the three different 3D chambers after 2 days in culture. (a) A TESM within its 50 $\mu$L 3D culture chamber right after the casting of the cell-laden hydrogel. (b) The same TESM after 2 days in culture under proliferation conditions: the fibrin-based hydrogel undergoes compaction around the pillars, (c, d) A 30 $\mu$L TESM inside a 3D chamber obtained from the ESCARGOT method before (c) and after (d) 2 days in culture, (e, f) 3D chambers fabricated with the Ecoflex Replica method provide equivalent results in terms of compaction of the cell-laden hydrogel. Scale bars, a-f, 1 mm.

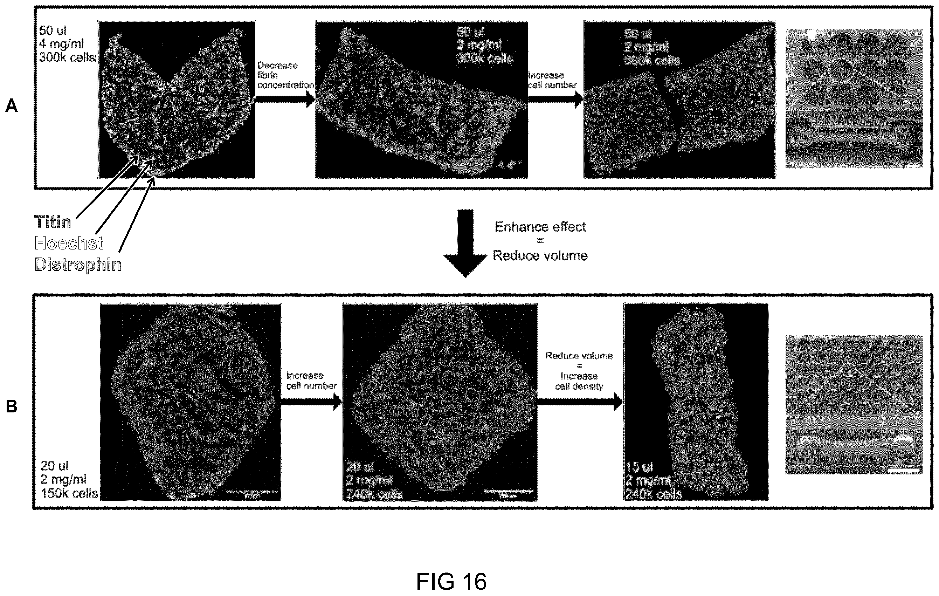

**FIG 16** illustrates optimization generating muscle in a muscle-on-chip device. Panel A: example of method optimization for muscle generated in a 50 ul-sized muscle-on-chip device. Increasing the cell density and decreasing the fibrin concentration resulted in enhanced density of muscle fibers. Antibody stainings: Titin is a sarcomeric marker indicative of maturation; Dystrophin is a cell membrane marker that should be around the titin-positive fibers; Hoechst stains nuclei (please note that muscle fibers should be multinucleated). Panel B: as above, but now for muscle generated in a 15-20 ul-sized muscle-on-chip device. The figure shows enhanced quality of muscle tissue by reducing the size of the device and by increasing the cell density.

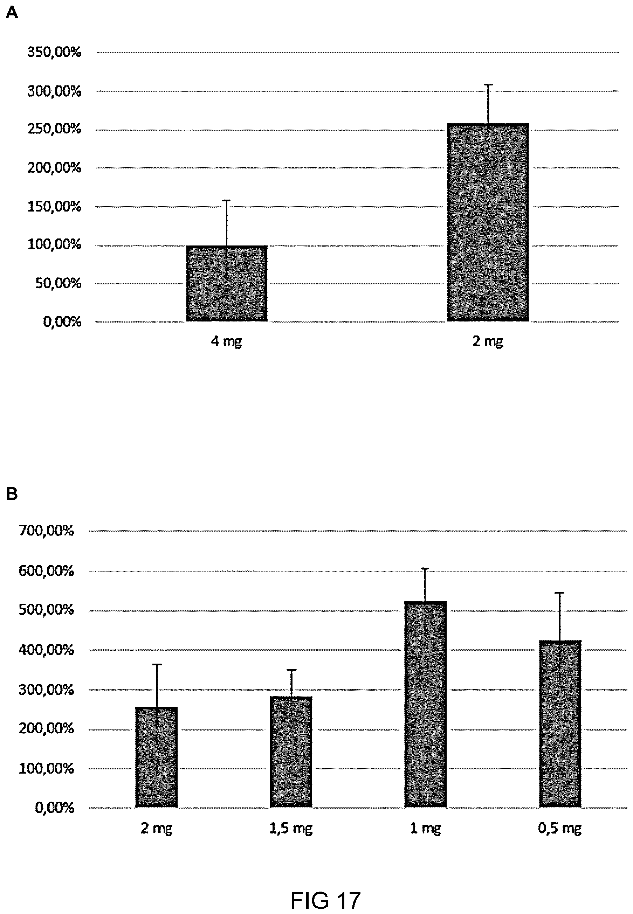

**FIG 17** illustrates effect of fibrin concentration on contractile force of in vitro cultured 3D skeletal muscle. On the X-axis, different concentrations of fibrin are shown (in mg/ml), on the Y axis the contractile force as % of the force at 4 mg/ml fibrin. The figure shows that the force at the commonly used fibrin concentration of 4 mg/ml results in the formation of muscle bundles with a force that is modest compared to muscle that was cultured at lower fibrin concentrations. The optimal fibrin concentration was 1 mg/ml, which resulted in a 5-fold increase in contractile force. The experiment was done in 2 parts, A and B.

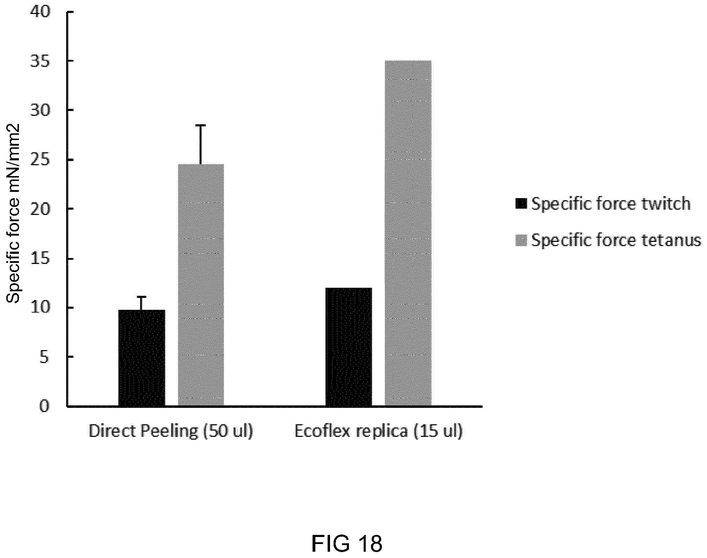

**FIG 18** illustrates the specific twitch force and specific tetanus force obtained with the "Direct Peeling Method" and the Ecoflex Replica Method". Control TESMs were either cultured in direct peeling (50 µl) or Ecoflex replica (15 µl) mold. After 7 days of differentiation TESMs were stimulated with a frequency of 1Hz (twitch) or 20 Hz (tetanus). Displacement of the pillar was recorded and specific force was calculated.

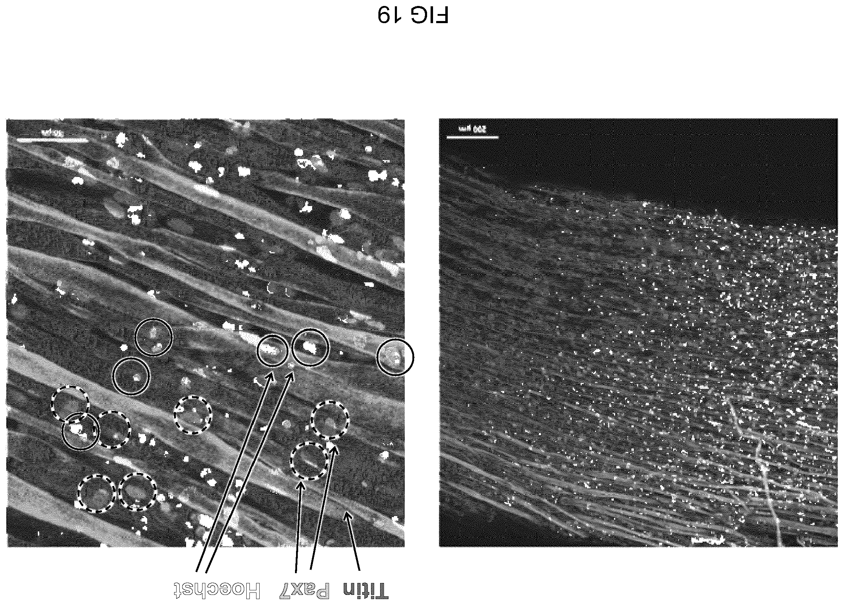

**FIG 19** illustrates an example of TESM after 7 days of differentiation. Immunofluorescent stainings were performed using antibodies against the sarcomeric protein titin (green - here recolored as dark grey), and the muscle stem cell marker Pax7 (red - here recolored as lighter gray, some indicated by dashed circles). Arrows indicate examples of stained Pax7 and nucleic. Hoechst (blue - here recolored as white, some indicated by solid line circles) staining to visualize nuclei. Note that the Pax7-positive cells are located adjacent to the myofibers in agreement with their natural location in skeletal muscle tissue. This is the first time that formation of Pax7-positive muscle stem cells has been demonstrated within TESMs that were derived from human iPSCs obtained in a transgene-free manner. Upon culturing in 2D on petridishes of the myogenic progenitors used to generate the TESMs, Pax7 expression is rapidly lost, going from 100% after FACS sorting to 3% after culture in 2D), as published by us previously (see Figure 5B in PMID: 29731431). The surprising result is the fact that upon culturing the same cells in a 3D environment in TESMs, they re-form large numbers of Pax7+ muscle stem cells. Therefore, the TESMs generated under the conditions employed in this application provide a novel tool for the *in vitro* generation of large numbers of transgene-free, human Pax7+ muscle stem cells that are anticipated to be useful for regenerative purposes.

As a specific example, 3D human Tissue Engineered Skeletal Muscles (TESMs) can be generated as follows.

Hydrogel generated as ECM for the 3D culture, composed of bovine fibrinogen (Sigma-Aldrich), is dissolved in DMEM high glucose (2 mg/mL final concentration), Matrigel growth factor reduced (20% v/v, Corning Life Sciences, Amsterdam, NL), thrombin from human plasma (Sigma-Aldrich) dissolved in 0.1 % BSA in PBS (1% v/v, 0.5 U/mL final concentration), myogenic progenitor proliferation medium (69% v/v). Matrigel, previously stored at -20°C, was kept at 4°C 2 hours before usage; fibrinogen, stored at -80°C, was kept at 4°C 1 hour before usage. Myogenic Progenitors used for the generation of the TESMs were harvested before passage 10. After detachment from culture dishes, cells were suspended in myogenic progenitor proliferation medium at a concentration of $16 \cdot 10^6$ cells/mL and the suspension then mixed with fibrinogen and Matrigel. Lastly, thrombin was added to the mix immediately before pipetting the cell-hydrogel mix into 3D chambers. The 3D chambers were manufactured according to the methods of manufacturing a microdevice as described herein.

The chambers were previously coated with Pluronic F-127 (Sigma-Aldrich) for 1 hour at room temperature, to prevent unwanted adhesion of the hydrogel. Before the casting of the hydrogel inside the 3D chambers, the Pluronic F-127 is preferably removed after its incubation of a minimum of 1 hour at RT. This can be an important step: as minuscule traces of Pluronic still present as a result of an improper aspiration can affect the generation of a TESM. Tiny bubbles can form in the interface between the traces of Pluronic and the cell-laden hydrogel after casting. This can negatively influence the subsequent solidification and compaction of the hydrogel into the bundle-like shape of a TESM, leading to poor structural integrity or incomplete formation of the engineered tissue.

A volume of 15 µL was pipetted for each TESM in the chambers. All the hydrogel components, as well as tubes and micropipette's tips were kept on ice prior and during the duration of the experiments. The final solution was then left to polymerize for 30 minutes in the incubator at 37°C and 5% CO2, before adding the TESM proliferation medium. This consisted in the myogenic progenitors proliferation medium supplemented with 6-aminocaproic acid (1.5 mg/mL, Sigma-Aldrich). After 48 hours, proliferation medium was switched to TESM differentiation medium, consisting of DMEM high glucose supplemented with 1% knock-out serum replacement, 1% ITS-X (all Gibco), 1% penicillin-G (Sigma-Aldrich), 1% Glutamax (Sigma-Aldrich), 6-aminocaproic acid (1.5 mg/mL, Sigma-Aldrich). Half volume of TESM differentiation medium was replaced every 48 hours. TESMs were kept on agitation at 55 rpm (Celltron orbital shaker, Infors HT, Switzerland) at 37 °C and 5% $CO_2$ for all the duration of the culture time.

Twitch and tetanic contractions were induced by electrical stimulation with either a frequency of 1 Hz (twitch) or 20 Hz (tetanus) at 2.5 V/cm with 10% duty cycle using a function generator. During stimulation the pillar displacement was captured with a fast camera recording at 60 frames per second. The height of the TESM on the pillar was measured for each replica and included in the calculation. Contractile force was calculated using Young's Elastic Modulus (E) of PDMS, the geometrical properties and the position of the TESM on the pillar (R, a and L) and displacement ($\delta$) of the PDMS pillar using the following *Force in N =* $\frac{6E\pi R^4}{4a^2(3L-a)}\delta$ formula [Legant et al. "Microfabricated tissue gauges to measure and manipulate forces from 3D microtissues", PNAS (25), 10097-10102 (2009)]. For the calculation of specific force paraffin based cross sections were generated from 2-3 TESMs and the specific force was calculated by dividing the force by cross sectional area. The results are shown in FIG 17 and table 1, which also shows a comparison with the specific twitch force and specific tetanus force that have been measured in skeletal muscle bundles generated so far.

Table 1. Comparison of specific force in human skeletal muscle bundles

| Study | Specific force twitch | Specific force tetanus | Cell source | Time of analysis after differentiation |
|---|---|---|---|---|
| Rao et al, 2018, DOI: 10.1038/s41467-017-02636-4 | ~0,8 mN/mm$^2$ | ~3 mN/mm$^2$ | hiPSC; Pax7 overexpression | 2 weeks |
| Madden et al, 2015, DOI: 10.7554/eLife. 04885 | ~6 mN/mm$^2$ | ~12 mN/mm$^2$ | Human primary Myoblasts | 2 weeks |
| Xu et al, 2019, DOI: 10.1002/adbi. 201900 005 | N.R. | ~33 mN/mm$^2$ | hiPSC; Pax7 overexpression | 4 weeks |
| Afshar et al, 2020, DOI: 10.1038/s41598-020-62837-8 | N.R. | ~5 mN/mm$^2$ | Human primary Myoblasts | 2 weeks |
| Selvaraj et al, 2020, DOI: 10.7554/eLife. 47970 | ~5 mN/mm2* | ~5 mN/mm2* | hiPSC; transgene-free | 5 days |
| Mills et al, 2019, DOI: 10.1016/j.biomaterial s.2018.11.030 | ~2 mN/mm$^2$ | ~4,9 mN/mm$^2$ | Human primary Myoblasts | 7 days |
| Direct Peeling 50 ul (this study) | ~10 mN/mm$^2$ | ~25 mN/mm$^2$ | hiPSC; transgene-free | 7 days |
| Ecoflex replica 15 ul (this study) | ~12 mN/mm$^2$ | ~35 mN/mm$^2$ | hiPSC; transgene-free | 7 days |
| N.R. Not Reported, * no specific force reported, calculation based on area of longitudinal staining. | | | | |

For the purpose of clarity and a concise description, features are described herein as part of the same or separate embodiments. While embodiments were shown for the manufacturing of specific microdevices using specific material and methods, also alternative ways may be envisaged by those skilled in the art having the benefit of the invention for achieving a similar function and result. For example, instead of 3D printing the first (positive) mold also other additive and/or subtractive manufacturing methods can be used. For example Jet fusion or selective laser sintering can be used. In principle, the positive first mold can be created by any process, such as injection molding, although this may be more difficult and less versatile. Alternatively, or in addition to stretching the elastically deformable negative mold structure it can also be envisaged to dissolve the negative mold structure. For example, the second material forming the negative mold structure can comprises or essentially consists of hydrogels, for example agarose, gelatin, or alginate. This may be less efficient because the negative mold can only be used once, but a new mold can be created from the positive, e.g. printed, first mold.

The various elements of the embodiments as discussed and shown offer certain advantages, such as the Generation of 3D human Tissue Engineered Skeletal Muscles. Of course, it is to be appreciated that any one of the above embodiments or processes may be combined with one or more other embodiments or processes to provide even further improvements in finding and matching designs and advantages. It is appreciated that this disclosure offers particular advantages to basic biological studies, 3D muscle-on-a-chip applications, drug screening, phase I-IV clinical trials, personalized medicine, and in general can be applied for any application wherein it is desired to easily manufacture and use microdevices or chips with reproducible sub-micron features. In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function.

**Claims**

1. A method of manufacturing a microstructure, the method comprising

   using a 3D printing process to form a positive mold structure of a printable, first material, wherein the positive mold structure is a positive of the microstructure to be manufactured;
   filling the positive mold structure with a second material to form an elastically deformable negative mold structure, wherein the second material has a lower Young's modulus than the first material;
   filling the negative mold structure with a third material to form the microstructure; and
   releasing the microstructure from the negative mold structure, wherein the negative mold structure is stretched to facilitate the release of the microstructure.

2. The method according to the preceding claim, wherein the microstructure comprises at least one micropillar with a first diameter less than a millimeter, wherein a top of the micropillar is provided with a cap having a second diameter that is larger than the first diameter of the micropillar on which the cap is provided by at least fifty percent.

3. The method according to the preceding claim, wherein the micropillar comprises a widening section below the cap wherein a diameter of the widening section gradually decreases in a downward direction from the second diameter of the cap to the first diameter of the micropillar below.

4. The method according to the preceding claim, wherein the microstructure comprises a set of two or more of the capped micropillars disposed in a microchamber with a maximum capacity between five and hundred microliters for holding liquid.

5. The method according to any of the preceding claims, wherein the second material forming the elastically deformable negative mold structure is more flexible than the first material forming the 3D printed positive mold structure and more flexible than the third material forming the microstructure, and wherein third material forming the microstructure is more flexible than the first material forming the 3D printed positive mold structure.

6. The method according to any of the preceding claims, wherein the second material forming the elastically deformable negative mold structure is reversibly stretchable by at least a factor three without breaking.

7. The method according to any of the preceding claims, wherein the second material forming the elastically deformable negative mold structure comprises or essentially consists of a silicone elastomer, and the microstructure is formed of a biocompatible elastomer, preferably a polymeric organosilicon compound such as polydimethylsiloxane (PDMS).

8. The method according to any of the preceding claims, wherein the 3D printing process comprises stereo-lithography, wherein the first material comprises a liquid polymeric resin that is solidified by a laser spot and/or light pattern

9. A lab-on-chip comprising microstructures obtainable by the method according to any of the preceding claims, wherein the microstructures comprise one or more microchambers, wherein a respective microchamber of the one or more microchambers has a maximum capacity between five and hundred microliter for holding liquid, wherein the respective microchamber comprises at least two micropillars, wherein a respective micropillar of the at least two micropillars has a first diameter less than a millimeter, wherein a top of the respective micropillar is provided with a cap having a second diameter that is larger than the first diameter of the micropillar below by at least fifty percent, wherein the respective micropillar comprises a widening section below the cap wherein a diameter of the widening section gradually decreases in a downward direction from the second diameter of the cap to the first diameter of the micropillar below, wherein the micropillars including the cap and widening section there between are integrally formed of an elastomeric material such as PDMS.

10. Use of the lab-on-chip according to the preceding claim for generating and/or analyzing a contractile and load-bearing tissue, such as tendons or muscle tissue, preferably 3D skeletal muscle tissue.

11. A method for generating muscle tissue, preferably skeletal muscle bundles and/or skeletal muscle stem cells, the method comprising

providing a microchamber of a lab-on-chip comprising microstructures with myogenic progenitor cells, a hydrogel, and a culture medium, ,and

culturing the myogenic progenitor cells to generate muscle tissue.

12. The method according to claim 11, wherein:

the hydrogel comprises 0.2-4 mg/ml, preferably 0.5-2 mg/ml, fibrinogen, extracellular matrix protein such as laminin, nidogen, collagen, elastin, fibronectin and/or heparan sulfate proteoglycans, myogenic progenitor proliferation medium, such as DMEM or DMEM high glucose, comprising antibiotics, fetal bovine serum (FBS) and fibroblast growth factor 2 (FGF2), preferably in a concentration of preferably 80-120 ng/ml, the hydrogel further optionally comprising thrombin, preferably human thrombin, preferably in an amount of 0.5-5% v/v, the myogenic progenitor cells are added to the microchamber in a concentration of $10^6$ - $10^9$ cells/ml, more preferably at a concentration of $10^7$ - $10^8$ cells/ml, and the culture medium is:

proliferation medium, such as DMEM or DMEM high glucose, comprising antibiotics, fetal bovine serum (FBS) and fibroblast growth factor 2 (FGF2), preferably in a concentration of preferably 80-120 ng/ml, comprising 6-aminocaproic acid, preferably in a concentration of 0.5-5 mg/ml, or aprotinin, preferably in a concentration of 60-100 $\mu$g/ml, or

differentiation medium, such as DMEM or DMEM high glucose, comprising antibiotics, ITS-X, preferably in a concentration of 0.5-2.5% v/v, knock-out serum replacement, preferably in a concentration of 0.5-2.5% v/v, L-glutamine and 6-aminocaproic acid, preferably in a concentration of 0.5-5 mg/ml, or aprotinin, preferably in a concentration of 60-100 $\mu$g/ml,

preferably whereby the culture medium is initially said proliferation medium and after 1.5-3 days is replaced by said differentiation medium.

13. A muscle tissue generated by the method according to claim 11 or 12, preferably wherein said muscle tissue is skeletal muscle bundles or muscle stem cells, more preferably wherein the skeletal muscle bundles have a specific twitch force *in vitro* of more than 7 mN/mm$^2$ and a specific tetanus force more than 33 mN/mm$^2$.

14. Use of a muscle tissue according to claim 13 for *in vitro* screening of a test compound or drug.

15. A muscle tissue according to claim 13 for use in therapy, preferably in regenerative therapy and/or in the treatment of a muscle disorder, preferably wherein said muscle disorder is a congenital muscle disease or congenital muscular dystrophy, more preferably wherein said muscle disorder is selected from the group consisting of Duchenne muscular dystrophy, Becker muscular dystrophy, myotonic dystrophy, limb-girdle dystrophy and facioscapulohumeral dystrophy, distal myopathies, myotonic syndromes, ion channel muscle diseases, malignant hyperthermias, metabolic myopathies, hereditary cardiomyopathies, congenital myasthenic syndromes, motor neuron diseases, hereditary ataxias, hereditary motor sensory neuropathies (HMSN), hereditary paraplegias, fibromyalgia, amyotrophic lateral sclerosis (ALS), myasthenia gravis and Pompe disease.

**FIG 1**

Design | Fabrication | Replica molding

a) Direct Peeling method
i) 1 mm Ø pillars
ii) FDM printing → Negative ABS mold
iii) PDMS Peeling → Positive PDMS : 50 µL chambers
iv) Pillar: no cap — 3.2 mm, 1 mm
v)

b) ESCARGOT method
i) 0.75 mm Ø pillars
ii) FDM printing → Negative ABS mold
iii) PDMS Acetone → Positive PDMS : 30 µL chambers
iv) Pillar: with cap — 3.2 mm, 0.75 mm, 1.2 mm
v)

c) Ecoflex Replica method
i) 0.5 mm Ø pillars
SLA printing → Coating & Ecoflex replica → Negative ecoflex mold
ii) Positive resin mold
Coating & PDMS → iii) Positive PDMS : 15 µL chambers
iv) Pillar: with cap — 3 mm, 0.5 mm, 1 mm
v)

FIG 2

FIG 3

FIG 4

FIG 5

FIG 6

## Direct Peeling method

3D CAD model

a)
FDM
3D Printing

3D printed ABS negative

b)
PDMS

Printed structure inside
6 cm plastic dish

c)
Remove from
dish

d)
Peeling

PDMS replica

FIG 7

EP 3 922 431 A1

### ESCARGOT method

FIG 8

27

## Ecoflex Replica method

3D CAD model

3D printed resin positive

Overnight coating

a) SLA 3D Printing

b) PFTOS Coating

c) Ecoflex

d) PFTOS Coating

f)

e) PDMS

PDMS replica

Stretching and Peeling

Overnight coating

FIG 9

EP 3 922 431 A1

FIG 10

3D printed resin structure

PDMS replica

a)

c)

b)

d)

FIG 11

30

FIG 12

FIG 13

FIG 14

a) 3D printed original structure

b) 3rd PDMS replica

c) 5th PDMS replica

d) 11th PDMS replica

e) 14th PDMS replica

FIG 15

Day 0 Day 2

a) b) Direct Peeling method 50 µL chambers

c) d) ESCARGOT method 30 µL chambers

e) f) Ecoflex Replica method 15 µL chambers

FIG 16

A

B

FIG 17

FIG 18

FIG 19

Europäisches
Patentamt

European
Patent Office

Office européen
des brevets

# EUROPEAN SEARCH REPORT

Application Number

EP 20 17 8818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BALMERT STEPHEN C ET AL: "Dissolving undercut microneedle arrays for multicomponent cutaneous vaccination", JOURNAL OF CONTROLLED RELEASE, ELSEVIER, AMSTERDAM, NL, vol. 317, 19 November 2019 (2019-11-19), pages 336-346, XP086001793, ISSN: 0168-3659, DOI: 10.1016/J.JCONREL.2019.11.023 [retrieved on 2019-11-19] | 1-3,5-8 | INV.<br>B29C39/00<br>C12M3/00<br>C12M1/32<br>G01N33/50<br>C12N5/077<br>C12N5/00 |
| Y | * the whole document *<br>* figures 2,3 *<br>* Materials and methods * | 1-15 | |
| X | OSAKI TATSUYA ET AL: "On-chip 3D neuromuscular model for drug screening and precision medicine in neuromuscular disease", NATURE PROTOCOLS, NATURE PUBLISHING GROUP, GB, vol. 15, no. 2, 13 January 2020 (2020-01-13), pages 421-449, XP037005236, ISSN: 1754-2189, DOI: 10.1038/S41596-019-0248-1 [retrieved on 2020-01-13] | 9-15 | |
| | | | TECHNICAL FIELDS SEARCHED (IPC)<br>B29C<br>C12M<br>G01N<br>C12N |
| Y | * the whole document *<br>* figures 2b, 4f *<br>* Experimental design * | 1-15 | |
| X | US 2014/220555 A1 (CHEN CHRISTOPHER S [US] ET AL) 7 August 2014 (2014-08-07) | 9-15 | |
| Y | * figures 1a, 2, 6, 7 *<br>* claims 1-45 * | 1-15 | |

-/--

The present search report has been drawn up for all claims

| Place of search | Date of completion of the search | Examiner |
|---|---|---|
| Munich | 5 October 2020 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 17 8818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/138132 A1 (UNIV ILLINOIS [US]; KING WILLIAM P [US]; CANNON ANDREW H [US]) 2 December 2010 (2010-12-02) * figure 1 * * claims 1-26 * ----- | 1-9 | |
| X,P | ALESSANDRO IULIANO ET AL: "Coupling 3D Printing and Novel Replica Molding for In House Fabrication of Skeletal Muscle Tissue Engineering Devices", ADVANCED MATERIALS TECHNOLOGIES, 26 July 2020 (2020-07-26), page 2000344, XP055735796, DE ISSN: 2365-709X, DOI: 10.1002/admt.202000344 * the whole document * * figures 1, 2, 5 * * Experimental Section * ----- | 1-15 | |

TECHNICAL FIELDS
SEARCHED    (IPC)

The present search report has been drawn up for all claims

| Place of search | Date of completion of the search | Examiner |
|---|---|---|
| Munich | 5 October 2020 | Bayer, Martin |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 17 8818

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2020

| Patent document cited in search report | | | Publication date | Patent family member(s) | | Publication date |
|---|---|---|---|---|---|---|
| US 2014220555 | A1 | | 07-08-2014 | EP 2766473 A1 | | 20-08-2014 |
| | | | | US 2014220555 A1 | | 07-08-2014 |
| | | | | WO 2013056019 A1 | | 18-04-2013 |
| WO 2010138132 | A1 | | 02-12-2010 | US 2012126458 A1 | | 24-05-2012 |
| | | | | WO 2010138132 A1 | | 02-12-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8058065 B
- US 8129187 B
- US 8211697 B
- US 8257941 B
- US 8278104 B
- US 8546140 B
- US 8791248 B
- US 9175268 B
- US 20080003560 A
- US 20080233610 A
- US 20100184227 A
- US 20130029866 A
- US 20130040302 A
- US 20130130387 A
- EP 2072618 A1
- WO 2009032194 A
- WO 2009032456 A
- WO 2010042490 A
- WO 2010077955 A
- WO 2017196175 A

### Non-patent literature cited in the description

- **LEGANT et al.** Microfabricated tissue gauges to measure and manipulate forces from 3D microtissues. *Proc. Natl. Acad. Sci.,* 2009, vol. 106
- **RAMADE et al.** Microfabrication of a Platform to Measure and Manipulate the Mechanics of Engineered Microtissues. *Methods Cell Biol.,* 2014, vol. 121, 191-211
- **KALMAN et al.** Quick and easy microfabrication of T-shaped cantilevers to generate arrays of microtissues. *Biomed. Microdevices,* 2016, vol. 18, 43
- **AGRAWAL et al.** Skeletal muscle-on-a-chip: an in vitro model to evaluate tissue formation and injury. *Lab Chip,* 2017, vol. 17, 3447-3461
- **OSAKI et al.** Microphysiological 3D model of amyotrophic lateral sclerosis (ALS) from human iPS-derived muscle cells and optogenetic motor neurons. *Sci. Adv.,* 2018, vol. 4, eaat5847
- **OSAKI et al.** On-chip 3D neuromuscular model for drug screening and precision medicine in neuromuscular disease. *Nat. Protoc.,* 2020, vol. 15, 421-449
- **TAKAHASHI et al.** Engineered Human Contractile Myofiber Sheets as a Platform for Studies of Skeletal Muscle Physiology. *Sci. Rep.,* 2018, vol. 8, 13932
- **RONALDSON-BOUCHARD et al.** Engineering of human cardiac muscle electromechanically matured to an adult-like phenotype. *Nat. Protoc.,* 2019, vol. 14, 2781-2817
- **CHRISTENSEN et al.** 3D Printed Hydrogel Multiassay Platforms for Robust Generation of Engineered Contractile Tissues. *Biomacromolecules,* 2020, vol. 21, 356-365
- **AFSHAR et al.** A 96-well culture platform enables longitudinal analyses of engineered human skeletal muscle microtissue strength. *bioRxiv,* 2019, 562819
- **SAGGIOMO et al.** Simple 3D Printed Scaffold-Removal Method for the Fabrication of Intricate Microfluidic Devices. *Advanced Science,* 2015, vol. 2, 1500125
- **LEGANT et al.** Microfabricated tissue gauges to measure and manipulate forces from 3D microtissues. *PNAS,* 2009, vol. 25, 10097-10102
- **HINDS et al.** The role of extracellular matrix composition in structure and function of bioengineered skeletal muscle. *Biomaterials,* 2011, vol. 32, 3575-3583